# EUROPEAN PATENT SPECIFICATION

(11) **EP 2 575 883 B1**
(45) Date of publication and mention of the grant of the patent: **04.12.2019**
(21) Application number: 11790443.3
(22) Date of filing: 03.06.2011
(51) Int. Cl.: A61K 39/395, C12N 15/113, A61K 38/17, A61K 45/06, A61K 31/7088

(54) **TREATMENT OF INFLAMMATORY DISORDERS**
BEHANDLUNG VON ENTZÜNDUNGSERKRANKUNGEN
TRAITEMENT DE TROUBLES INFLAMMATOIRES

(30) Priority: 04.06.2010 US 351505 P
(43) Date of publication of application: 10.04.2013
(73) Proprietor: The Brigham and Women's Hospital, Inc., Boston, MA 02115 (US)
(72) Inventor: BRENNER, Michael, Barry, Newton, MA 02458 (US); NOSS, Erika, Heidi, West Roxbury, MA 02132 (US); CHANG, Sook, Kyung, Dongan-gu, Aryang Gyeonggi-do 14102 (KR)
(74) Representative: Boult Wade Tennant LLP
(86) International application number: PCT/US2011/039002
(87) International publication number: WO 2011/153397

(56) References cited:
- WO-A1-2011/140173
- WO-A2-2009/101059
- WO-A2-2009/101059
- WO-A2-2010/091384
- US-A1- 2005 002 919
- US-A1- 2005 215 482
- US-A1- 2008 214 487
- Jeffry A. Katz: "The Practical Use of Corticosteroids in the Treatment of Inflammatory Bowel Disease", , 1 January 2005 (2005-01-01), pages 14-25, XP055091963, Retrieved from the Internet: URL:http://www.practicalgastro.com/pdf/Kat zArticleJan05.pdf [retrieved on 2013-12-06]
- MONAHAN T S ET AL: "A novel function for cadherin 11/osteoblast-cadherin in vascular smooth muscle cells: Modulation of cell migration and proliferation", JOURNAL OF VASCULAR SURGERY, C.V. MOSBY CO., ST. LOUIS, MO, US, vol. 45, no. 3, 1 March 2007 (2007-03-01), pages 581-589, XP022689755, ISSN: 0741-5214, DOI: 10.1016/J.JVS.2006.12.016 [retrieved on 2007-02-22]
- COSTELLO ET AL.: 'Dissection of the inflammatory bowel disease transcriptome using genome- wide cDNA microarrays.' PLOS MED vol. 2, no. 8, August 2005, pages 0771 - 0787, XP055070528
- Jose Miguel López-Novoa ET AL: "Inflammation and EMT: an alliance towards organ fibrosis and cancer progression", EMBO molecular medicine, vol. 1, no. 6-7, 1 September 2009 (2009-09-01), pages 303-314, XP055325554, Weinheim ISSN: 1757-4676, DOI: 10.1002/emmm.200900043
- COUSSENS L M ET AL: "Inflammation and cancer", NATURE, NATURE PUBLISHING GROUP, UNITED KINGDOM, vol. 420, no. 6917, 1 January 2002 (2002-01-01), pages 860-867, XP002356258, ISSN: 0028-0836, DOI: 10.1038/NATURE01322
- Anne K. Farina ET AL: "Post-Transcriptional Regulation of Cadherin-11 Expression by GSK-3 and [beta]-Catenin in Prostate and Breast Cancer Cells", PLoS ONE, vol. 4, no. 3, 10 March 2009 (2009-03-10), page e4797, XP055414996, DOI: 10.1371/journal.pone.0004797
- Brendan J R Whittle ET AL: "Reduction of experimental colitis in the rat by inhibitors of glycogen synthase kinase-3 [beta]", BRITISH JOURNAL OF PHARMACOLOGY, vol. 147, no. 5, 1 March 2006 (2006-03-01) , pages 575-582, XP055414985, UK ISSN: 0007-1188, DOI: 10.1038/sj.bjp.0706509

### SEQUENCE LISTING

<110> The Brigham and Women's Hospital, Inc. Brenner, Michael B. Noss, Erika H. Chang, Sook K.
<120> TREATMENT OF INFLAMMATORY DISORDERS
<130> B0801.70368WO00
<150> US 61/351,505
   <151> 2010-06-04
<160> 7
<170> PatentIn version 3.5
<210> 1
   <211> 3654
   <212> DNA
   <213> H. sapiens
<400> 1
   agatgccgcg ggggccgctc gcagccgccg ctgacttgtg aatgggaccg ggactggggc      60
   cgggactgac accgcagcgc ttgccctgcg ccagggactg gcggctcgga ggttgcgtcc     120
   accctcaagg gccccagaaa tcactgtgtt ttcagctcag cggccctgtg acattccttc     180
   gtgttgtcat ttgttgagtg accaatcaga tgggtggagt gtgttacaga aattggcagc     240
   aagtatccaa tgggtgaaga agaagctaac tggggacgtg ggcagccctg acgtgatgag     300
   ctcaaccagc agagacattc catcccaaga gaggtctgcg tgacgcgtcc gggaggccac     360
   cctcagcaag accaccgtac agttggtgga aggggtgaca gctgcattct cctgtgccta     420
   ccacgtaacc aaaaatgaag gagaactact gtttacaagc cgccctggtg tgcctgggca     480
   tgctgtgcca cagccatgcc tttgccccag agcggcgggg gcacctgcgg ccctccttcc     540
   atgggcacca tgagaagggc aaggaggggc aggtgctaca gcgctccaag cgtggctggg     600
   tctggaacca gttcttcgtg atagaggagt acaccgggcc tgaccccgtg cttgtgggca     660
   ggcttcattc agatattgac tctggtgatg ggaacattaa atacattctc tcaggggaag     720
   gagctggaac catttttgtg attgatgaca aatcagggaa cattcatgcc accaagacgt     780
   tggatcgaga agagagagcc cagtacacgt tgatggctca ggcggtggac agggacacca     840
   atcggccact ggagccaccg tcggaattca ttgtcaaggt ccaggacatt aatgacaacc     900
   ctccggagtt cctgcacgag acctatcatg ccaacgtgcc tgagaggtcc aatgtgggaa     960
   cgtcagtaat ccaggtgaca gcttcagatg cagatgaccc cacttatgga aatagcgcca    1020
   agttagtgta cagtatcctc gaaggacaac cctatttttc ggtggaagca cagacaggta    1080
   tcatcagaac agccctaccc aacatggaca gggaggccaa ggaggagtac cacgtggtga    1140
   tccaggccaa ggacatgggt ggacatatgg gcggactctc agggacaacc aaagtgacga    1200
   tcacactgac cgatgtcaat gacaacccac caaagtttcc gcagagcgta taccagatgt    1260
   ctgtgtcaga agcagccgtc cctggggagg aagtaggaag agtgaaagct aaagatccag 1320
   acattggaga aaatggctta gtcacataca atattgttga tggagatggt atggaatcgt 1380
   ttgaaatcac aacggactat gaaacacagg agggggtgat aaagctgaaa aagcctgtag 1440
   attttgaaac caaaagagcc tatagcttga aggtagaggc agccaacgtg cacatcgacc 1500
   cgaagtttat cagcaatggc cctttcaagg acactgtgac cgtcaagatc tcagtagaag 1560
   atgctgatga gccccctatg ttcttggccc caagttacat ccacgaagtc caagaaaatg 1620
   cagctgctgg caccgtggtt gggagagtgc atgccaaaga ccctgatgct gccaacagcc 1680
   cgataaggta ttccatcgat cgtcacactg acctcgacag atttttcact attaatccag 1740
   aggatggttt tattaaaact acaaaacctc tggatagaga ggaaacagcc tggctcaaca 1800
   tcactgtctt tgcagcagaa atccacaatc ggcatcagga agccaaagtc ccagtggcca 1860
   ttagggtcct tgatgtcaac gataatgctc ccaagtttgc tgccccttat gaaggtttca 1920
   tctgtgagag tgatcagacc aagccacttt ccaaccagcc aattgttaca attagtgcag 1980
   atgacaagga tgacacggcc aatggaccaa gatttatctt cagcctaccc cctgaaatca 2040
   ttcacaatcc aaatttcaca gtcagagaca accgagataa cacagcaggc gtgtacgccc 2100
   ggcgtggagg gttcagtcgg cagaagcagg acttgtacct tctgcccata gtgatcagcg 2160
   atggcggcat cccgcccatg agtagcacca acaccctcac catcaaagtc tgcgggtgcg 2220
   acgtgaacgg ggcactgctc tcctgcaacg cagaggccta cattctgaac gccggcctga 2280
   gcacaggcgc cctgatcgcc atcctcgcct gcatcgtcat tctcctggtc attgtagtat 2340
   tgtttgtgac cctgagaagg caaaagaaag aaccactcat tgtctttgag gaagaagatg 2400
   tccgtgagaa catcattact tatgatgatg aaggggtggg ggaagaagac acagaagcct 2460
   ttgatattgc caccctccag aatcctgatg gtatcaatgg atttatcccc cgcaaagaca 2520
   tcaaacctga gtatcagtac atgcctagac ctgggctccg gccagcgccc aacagcgtgg 2580
   atgtcgatga cttcatcaac acgagaatac aggaggcaga caatgacccc acggctcctc 2640
   cttatgactc cattcaaatc tacggttatg aaggcagggg ctcagtggcc gggtccctga 2700
   gctccctaga gtcggccacc acagattcag acttggacta tgattatcta cagaactggg 2760
   gacctcgttt taagaaacta gcagatttgt atggttccaa agacactttt gatgacgatt 2820
   cttaacaata acgatacaaa tttggcctta agaactgtgt ctggcgttct caagaatcta 2880
   gaagatgtgt aaacaggtat ttttttaaat caaggaaagg ctcatttaaa acaggcaaag 2940
   ttttacagag aggatacatt taataaaact gcgaggacat caaagtggta aatactgtga 3000
   aatacctttt ctcacaaaaa ggcaaatatt gaagttgttt atcaacttcg ctagaaaaaa 3060
   aaaacacttg gcatacaaaa tatttaagtg aaggagaagt ctaacgctga actgacaatg 3120
   aagggaaatt gtttatgtgt tatgaacatc caagtctttc ttctttttta agttgtcaaa 3180
   gaagcttcca caaaattaga aaggacaaca gttctgagct gtaatttcgc cttaaactct    3240
   ggacactcta tatgtagtgc atttttaaac ttgaaatata taatattcag ccagcttaaa    3300
   cccatacaat gtatgtacaa tacaatgtac aattatgtct cttgagcatc aatcttgtta    3360
   ctgctgattc ttgtaaatct ttttgcttct actttcatct taaactaata cgtgccagat    3420
   ataactgtct tgtttcagtg agagacgccc tatttctatg tcatttttaa tgtatctatt    3480
   tgtacaattt taaagttctt attttagtat acgtataaat atcagtattc tgacatgtaa    3540
   gaaaatgtta cggcatcaca cttatatttt atgaacattg tactgttgct ttaatatgag    3600
   cttcaatata agaagcaatc tttgaaataa aaaaagattt ttttttaaaa aaaa          3654
<210> 2
   <211> 796
   <212> PRT
   <213> H. sapiens
<400> 2
   Met Lys Glu Asn Tyr Cys Leu Gln Ala Ala Leu Val Cys Leu Gly Met
   1               5                   10                  15
   Leu Cys His Ser His Ala Phe Ala Pro Glu Arg Arg Gly His Leu Arg
               20                  25                  30
   Pro Ser Phe His Gly His His Glu Lys Gly Lys Glu Gly Gln Val Leu
           35                  40                  45
   Gln Arg Ser Lys Arg Gly Trp Val Trp Asn Gln Phe Phe Val Ile Glu
       50                  55                  60
   Glu Tyr Thr Gly Pro Asp Pro Val Leu Val Gly Arg Leu His Ser Asp
   65                  70                  75                  80
   Ile Asp Ser Gly Asp Gly Asn Ile Lys Tyr Ile Leu Ser Gly Glu Gly
                   85                  90                  95
   Ala Gly Thr Ile Phe Val Ile Asp Asp Lys Ser Gly Asn Ile His Ala
               100                 105                 110
   Thr Lys Thr Leu Asp Arg Glu Glu Arg Ala Gln Tyr Thr Leu Met Ala
           115                 120                 125
   Gln Ala Val Asp Arg Asp Thr Asn Arg Pro Leu Glu Pro Pro Ser Glu
       130                 135                 140
   Phe Ile Val Lys Val Gln Asp Ile Asn Asp Asn Pro Pro Glu Phe Leu
   145                 150                 155                 160
   His Glu Thr Tyr His Ala Asn Val Pro Glu Arg Ser Asn Val Gly Thr
                   165                 170                 175
   Ser Val Ile Gln Val Thr Ala Ser Asp Ala Asp Asp Pro Thr Tyr Gly
               180                 185                 190
   Asn Ser Ala Lys Leu Val Tyr Ser Ile Leu Glu Gly Gln Pro Tyr Phe
           195                 200                 205
   Ser Val Glu Ala Gln Thr Gly Ile Ile Arg Thr Ala Leu Pro Asn Met
       210                 215                 220
   Asp Arg Glu Ala Lys Glu Glu Tyr His Val Val Ile Gln Ala Lys Asp
   225                 230                 235                 240
   Met Gly Gly His Met Gly Gly Leu Ser Gly Thr Thr Lys Val Thr Ile
                   245                 250                 255
   Thr Leu Thr Asp Val Asn Asp Asn Pro Pro Lys Phe Pro Gln Ser Val
               260                 265                 270
   Tyr Gln Met Ser Val Ser Glu Ala Ala Val Pro Gly Glu Glu Val Gly
           275                 280                 285
   Arg Val Lys Ala Lys Asp Pro Asp Ile Gly Glu Asn Gly Leu Val Thr
       290                 295                 300
   Tyr Asn Ile Val Asp Gly Asp Gly Met Glu Ser Phe Glu Ile Thr Thr
   305                 310                 315                 320
   Asp Tyr Glu Thr Gln Glu Gly Val Ile Lys Leu Lys Lys Pro Val Asp
                   325                 330                 335
   Phe Glu Thr Lys Arg Ala Tyr Ser Leu Lys Val Glu Ala Ala Asn Val
               340                 345                 350
   His Ile Asp Pro Lys Phe Ile Ser Asn Gly Pro Phe Lys Asp Thr Val
           355                 360                 365
   Thr Val Lys Ile Ser Val Glu Asp Ala Asp Glu Pro Pro Met Phe Leu
       370                 375                 380
   Ala Pro Ser Tyr Ile His Glu Val Gln Glu Asn Ala Ala Ala Gly Thr
   385                 390                 395                 400
   Val Val Gly Arg Val His Ala Lys Asp Pro Asp Ala Ala Asn Ser Pro
                   405                 410                 415
   Ile Arg Tyr Ser Ile Asp Arg His Thr Asp Leu Asp Arg Phe Phe Thr
               420                 425                 430
   Ile Asn Pro Glu Asp Gly Phe Ile Lys Thr Thr Lys Pro Leu Asp Arg
           435                 440                 445
   Glu Glu Thr Ala Trp Leu Asn Ile Thr Val Phe Ala Ala Glu Ile His
       450                 455                 460
   Asn Arg His Gln Glu Ala Lys Val Pro Val Ala Ile Arg Val Leu Asp
   465                 470                 475                 480
   Val Asn Asp Asn Ala Pro Lys Phe Ala Ala Pro Tyr Glu Gly Phe Ile
                   485                 490                 495
   Cys Glu Ser Asp Gln Thr Lys Pro Leu Ser Asn Gln Pro Ile Val Thr
               500                 505                 510
   Ile Ser Ala Asp Asp Lys Asp Asp Thr Ala Asn Gly Pro Arg Phe Ile
           515                 520                 525
   Phe Ser Leu Pro Pro Glu Ile Ile His Asn Pro Asn Phe Thr Val Arg
       530                 535                 540
   Asp Asn Arg Asp Asn Thr Ala Gly Val Tyr Ala Arg Arg Gly Gly Phe
   545                 550                 555                 560
   Ser Arg Gln Lys Gln Asp Leu Tyr Leu Leu Pro Ile Val Ile Ser Asp
                   565                 570                 575
   Gly Gly Ile Pro Pro Met Ser Ser Thr Asn Thr Leu Thr Ile Lys Val
               580                 585                 590
   Cys Gly Cys Asp Val Asn Gly Ala Leu Leu Ser Cys Asn Ala Glu Ala
           595                 600                 605
   Tyr Ile Leu Asn Ala Gly Leu Ser Thr Gly Ala Leu Ile Ala Ile Leu
       610                 615                 620
   Ala Cys Ile Val Ile Leu Leu Val Ile Val Val Leu Phe Val Thr Leu
   625                 630                 635                 640
   Arg Arg Gln Lys Lys Glu Pro Leu Ile Val Phe Glu Glu Glu Asp Val
                   645                 650                 655
   Arg Glu Asn Ile Ile Thr Tyr Asp Asp Glu Gly Gly Gly Glu Glu Asp
               660                 665                 670
   Thr Glu Ala Phe Asp Ile Ala Thr Leu Gln Asn Pro Asp Gly Ile Asn
           675                 680                 685
   Gly Phe Ile Pro Arg Lys Asp Ile Lys Pro Glu Tyr Gln Tyr Met Pro
   690 695 700
   Arg Pro Gly Leu Arg Pro Ala Pro Asn Ser Val Asp Val Asp Asp Phe
   705 710 715 720
   Ile Asn Thr Arg Ile Gln Glu Ala Asp Asn Asp Pro Thr Ala Pro Pro
   725 730 735
   Tyr Asp Ser Ile Gln Ile Tyr Gly Tyr Glu Gly Arg Gly Ser Val Ala
   740 745 750
   Gly Ser Leu Ser Ser Leu Glu Ser Ala Thr Thr Asp Ser Asp Leu Asp
   755 760 765
   Tyr Asp Tyr Leu Gln Asn Trp Gly Pro Arg Phe Lys Lys Leu Ala Asp
   770 775 780
   Leu Tyr Gly Ser Lys Asp Thr Phe Asp Asp Asp Ser
   785 790 795
<210> 3
   <211> 34
   <212> PRT
   <213> H. sapiens
<400> 3
   Gly Trp Val Trp Asn Gln Phe Phe Val Ile Glu Glu Tyr Thr Gly Pro
   1 5 10 15
   Asp Pro Val Leu Val Gly Arg Leu His Ser Asp Ile Asp Ser Gly Asp
   20 25 30
   Gly Asn
<210> 4
   <211> 18
   <212> DNA
   <213> H. sapiens
<400> 4
   gaggcctaca ttctgaac 18
<210> 5
   <211> 6
   <212> PRT
   <213> H. sapiens
<400> 5
   Glu Ala Tyr Ile Leu Asn
   1 5
<210> 6
   <211> 6
   <212> PRT
   <213> artificial sequence
<220>
   <223> Fc fusion altered sequence
<400> 6
   Glu Ala Tyr Leu Glu Leu
   1 5
<210> 7
   <211> 18
   <212> DNA
   <213> artificial sequence
<220>
   <223> Fc fusion altered sequence
<400> 7
   gaggcctacc tcgagctc 18

## Description

### FIELD OF THE INVENTION

This invention relates to a cadherin-11 antibody, or antigen binding fragment thereof, or a cadherin-11 nucleic acid antagonist for use in methods and compositions for the treatment of inflammatory disorders, and more specifically non-joint inflammatory disorders. The methods involve administering a cadherin-11 antagonist to a subject to modulate cadherin-11 function, particularly in fibroblasts. The invention is defined by the claims and any other aspects or embodiments set forth herein not falling within the scope of the claims are for information only.

### BACKGROUND OF THE INVENTION

The adhesive interactions between cells and between cells and the extracellular matrix may be in involved in a wide variety of processes including, for example, modulation of the immune system, regulation of developmental processes and tumor progression and metastasis. These interactions are mediated by adhesion molecules which transduce information from the extracellular to the intracellular matrix.

Four families of adhesion molecules which mediate these interactions have been identified: the integrins, the cadherins, the selectins, and immunoglobulin-related molecules. In general, adhesion molecules are transmembrane proteins which contain an extracellular domain for interacting with an extracellular matrix or cellular component, a transmembrane domain spanning the cell membrane and a cytoplasmic domain for interacting with one or more cytoskeletal or cytoplasmic components.

The cadherins play an important role in the establishment and maintenance of intercellular connections between cells of the same type (reviewed in Geiger B. et al. (1992) Annual Review of Cell Biology 8:307; Kemler R. (1993) Trends in Gastroenterology 9:317; Takeichi M. (1990) Annual Review of Biochem. 59:237; Takeichi M. (1991) Science 251:1451). Cadherins are a superfamily of structurally related molecules that function in Ca⁺²-dependent homophilic adhesion. Cadherins are expressed on cells that form solid tissues, and are responsible for segregating and sorting cells during embryogenesis, establishing cell polarity, and maintaining tissue morphology. Structurally, cadherins are single chain polypeptides that are synthesized as precursors and cleaved during post-translational processing. They have large extracellular regions made up of 5 homologous domains, a single transmembrane segment and a cytoplasmic tail.

The cadherins are synthesized as precursors that are cleaved during post-translational processing. The mature cadherins are single chain molecules which include a relatively large extracellular domain (typically divided into five sections or "ectodomains"), a single transmembrane region and a cytoplasmic tail. Among the classical cadherins (i.e., P-(placenta), E- (epithelial), and N- (neural) cadherin), the cytoplasmic domain contains the highest degree of homology. The high degree of homology observed for the cytoplasmic domain reportedly is a reflection of the association of cadherins with a group of intracellular proteins, called catenins, that stabilize cadherin active conformation (Kemler R. (1993) Trends in Gastroenterology 9:317). It is generally believed that sequences in the extracellular domain are necessary to mediate homophilic (i.e., cadherin-to-cadherin) binding. A review of the literature indicates that research directed to understanding cadherin-mediated adhesion has focussed on efforts to elucidate the mechanism underlying cadherin-mediated homophilic cell adhesion. Little attention has been directed to understanding what, if any, role is played by cadherins in heterophilic adhesion. While it has been known for some time that integrins and other adhesion molecules function in immune system modulation, e.g., by playing a role in the adhesion of peripheral lymphocytes to endothelium and in homing to lymph nodes, relatively little is known regarding the mechanism by which lymphocytes home and transmigrate through the vascular endothelium to specifically target certain tissue locations.

The most highly conserved sequence shared by cadherins lies within the cytoplasmic domain. It is this region that mediates interaction with the cytoplasmic catenins proteins (Hirano S. et al. Cell 70:293-301, 1992). The presence of the cytoplasmic domain is essential to functioning of the cadherin as deletions in this region abolish catenin binding as well as cell-to-cell adhesion (Hulsken J, et al. J Cell Biol 127:1375-80, 1994) . The catenins (α, 102 kDa; β, 88-93 kDa, and γ, 80-83 kDa) begin to associate with cadherins almost immediately upon biosynthesis in a stable manner that is not disrupted in TX-100 detergent (Takeichi M. Curr Opin Cell Biol 7:619-27, 1995) and are thought to mediate anchorage of cadherins to the cytoskeleton (Yap AS. et al. Annu Rev Cell Dev Biol 13:11946, 1997). Functionally, α -catenin is necessary for cadherin mediated homophilic adhesion. Tumor cells expressing E-cadherin at the cell surface, but lacking α-catenin expression, fail to form cell-to-cell contacts unless α-catenin expression is restored through transfection (Chen H. et al. J Cell Sci 1141345-56, 1997; and Knudsen KA. et al. J Cell Biol 130:67-77, 1995). β-catenin is homologous to the Drosophila segment polarity protein armadillo as well as the cadherin associated protein plakoglobin. Plakoglobin, also termed γ-catenin, interacts more weakly with the cadherin/catenin complex, and is not always seen in cadherin precipitates.

A number of newly identified cadherin cDNA clones have been isolated using consensus oligonucleotides corresponding to cytoplasmic domain sequences that are highly conserved among cadherins and PCR cloning (Suzuki S. et al. Cell Reg 2:261-70, 1991).

Although cadherin function classically involves homophilic cell-to-cell adhesion (i.e., E-cadherin binds E-cadherin typically on another cell of the same type), however, murine E-cadherin expressed on epidermal keratinocytes also mediates adhesion to E-cadherin of Langerhans cells (Tang A. et al. Nature 361:82-5, 1993). Another counter-receptor for E-cadherin, namely the integrin α^{E}β₇ which is expressed on intraepithelial T cells (Cepek KL. et al. Nature 372:190-3, 1994 and U.S. Patent No. 5,610,281), has also been identified. This represents an example of heterophilic binding of E-cadherin to the α^{E}β₇ integrin counter-receptor.

### SUMMARY OF THE INVENTION

The invention provides a cadherin-11 antibody, or antigen-binding fragment thereof, or a cadherin-11 nucleic acid antagonist for use in methods and compositions for the treatment (including prevention) of inflammatory disorders, and particularly inflammatory disorders that are not inflammatory joint disorders (referred to herein as non-joint inflammatory disorders, or NJID). The inflammatory disorder may be an autoimmune disorder although it is not so limited. Disorders to be treated according to the invention therefore include but are not limited to inflammatory bowel disease (e.g., colitis, including severe colitis, ulcerative colitis, Crohn's disease), psoriasis, Graves opthalmopathy, various types of vasculitis, eczema such as atopic dermatitis, multiple sclerosis, atrial myxoma, inflammation associated with solid organ transplantation (various types of tissues), glomerulonephritis, interstitial nephritis, peritoneal inflammation and diverticulitis (and scarring) post surgical, post performation and/or post infection, pleural inflammation (and scarring) post surgical and/or post trauma such as blood in pleural space and post infection, as well as inflammation associated with burns (which also can lead to scarring).

Although not intending to be bound by any particular theory, it is postulated that cadherin-11 expression in fibroblasts can be targeted in order to reduce, among other things, the production of cytokines and growth factors, particularly those associated with inflammation from such fibroblasts. The target fibroblasts upregulate cadherin-11 expression and thus can be targeted by modulating, preferably antagonizing, cadherin-11 function. While inflammation is known to involve lymphocytes, and particularly activated lymphocytes, fibroblasts are not classically considered inflammatory cells. The antagonists of the invention can interfere with the ability of fibroblasts to make cytokines such as IL-6, chemokines such as MCP-1 and IL-8, prostaglandins such as PGE2, as well as other inflammatory factors. In some instances, the cadherin-11 antagonists inhibit partially or completely Th2 and/or Thl7 immune responses (e.g., by reducing the production and/or secretion of Th2 and/or Thl7 cytokines such as IL-4, IL-13 and IL-17). In some instances, the cadherin-11 antagonists inhibit partially or completely eosinophil responses (e.g., by reducing the levels of eosinophils recruited to a site of inflammation or by reducing the production and/or secretion of chemokines such as eotaxin including eotaxin-1). Again, although not intending to be bound by any particular mechanism, in part, it is the ability of the antagonists of the invention to block production of cytokines, chemokines and/or lipid mediators that renders them useful in inhibiting fibroblast inflammatory function and thus the ensuing inflammation, in some aspects and embodiments of the invention.

According to one aspect of the disclosure, a method is provided for treating a subject having an inflammatory disorder that is not a inflammatory joint disorder (i.e., the disorder is a non-joint inflammatory disorder or a NJID). The method involves administering to a subject in need of such treatment a therapeutically effective amount of a cadherin-11 antagonist. A cadherin-11 antagonist is an agent that interferes with a cadherin-11 function. Such interference may lead to partial or complete inhibition of cadherin-11 function. For the purposes of the invention, an important cadherin-11 function is growth factor or cytokine production, preferably from fibroblasts associated with NJID. It is to be understood, however, that a cadherin-11 antagonist may interfere with other cadherin-11 functions, including but not limited to cell-cell adhesion, cell-extracellular matrix adhesion, and apoptosis. In preferred embodiments, the subject is a human. Preferably, the subjects do not have an inflammatory joint disorder, such as arthritis. In some embodiments, the subject does not have abnormal cadherin-11 mediated adhesion occurring in the liver or the brain. Cadherin-11 mediated adhesion is the binding of cadherin-11 to its counter-receptor and such counter-receptor may be another cadherin-11 or another molecule altogether.

In one embodiment, the NJID is an autoimmune disorder. In one embodiment, the NJID is inflammatory bowel disease, such as colitis and including severe colitis, ulcerative colitis, or Crohn's disease. In one embodiment, the NJID is psoriasis. In one embodiment, the NJID is Graves opthalmopathy. In one embodiment, the NJID is vasculitis (i.e., inflammation of the blood vessels). In one embodiment, the NJID is eczema. In one embodiment, the NJID is atopic dermatitis. In one embodiment, the NJID is multiple sclerosis. In one embodiment, the NJID is atrial myxoma. In one embodiment, the NJID is inflammation resulting from solid organ transplantation. In one embodiment, the NJID is glomerulonephritis. In one embodiment, the NJID is interstitial nephritis. In one embodiment, the NJID is inflammation associated with a burn or other skin lesion. In one embodiment, the NJID is peritoneal inflammation and diverticulitis that occurs post-surgery, post-performation, or post-infection. In one embodiment, the NJID is pleural inflammation that occurs post-surgery, post-trauma, or post-infection.

In another aspect, the disclosure provides a method for preventing fibrosis in a subject by administering to a subject at risk of developing fibrosis a cadherin-11 antagonist. Again, although not intending to be bound by any particular mechanism, it is believed that in certain instances reduction or inhibition of an inflammatory reaction can prevent subsequent fibrosis. Accordingly, in some instances the subject manifests no symptoms of fibrosis (e.g., scarring).

The cadherin-11 antagonist may be administered systemically, including for example intravenously. In some embodiments, the cadherin-11 may be administered via a stent located in a blood vessel in a subject. This embodiment is particularly suited for the treatment of vasculitis. The stent may be located near the heart in order to treat atrial myxoma. In some embodiments, the cadherin-11 antagonist is administered locally including to the gut, the skin, or the eyes. The cadherin-11 antagonist may also be administered to a subject at the site of tissue or organ transplantation. The tissue or organ may be perfused with the cadherin-11 antagonist, or the cavity into which the tissue or organ will be placed can be washed with a cadherin-11 antagonist. Alternatively or additionally, the cadherin-11 antagonist may be administered systemically as well in transplant recipients.

In still other embodiments, the antagonist can be administered orally, topically, via a pulmonary route of administration, nasally, transdermally, subcutaneously or intravenously. In some instances, the cadherin-11 antagonist is administered by a systemic route such as a subcutaneous or intravenous route regardless of the nature of the inflammatory disorder.

In one embodiment, the cadherin-11 antagonist is a cadherin-11 binding peptide. In one embodiment, the cadherin-11 binding peptide is an anti-cadherin-11 antibody or an antigen-binding antibody fragment. In one instance, the cadherin-11 binding peptide is a cadherin-11 fusion protein. In one instance, the cadherin-11 binding peptide comprises full length cadherin or a fragment thereof.

In one embodiment, the cadherin-11 antagonist is a cadherin-11 nucleic acid antagonist. In one embodiment, the cadherin-11 nucleic acid antagonist is a cadherin-11 siRNA. In one embodiment, the cadherin-11 nucleic acid antagonist is a cadherin-11 ribozyme. In one embodiment, the cadherin-11 nucleic acid antagonist is a cadherin-11 antisense molecule. In one embodiment, cadherin-11 nucleic acid antagonist is a nucleic acid encoding full length cadherin-11 or a fragment thereof. In one embodiment, cadherin-11 nucleic acid antagonist is an aptamer.

In one instance, the cadherin-11 antagonist is a small molecule.

In some instances, the cadherin-11 antagonists are antibodies while in other embodiments they are not antibodies. Antibodies may be monoclonal antibodies, polyclonal antibodies, chimeric antibodies including humanized antibodies, camelids, single chain antibodies, and the like.

The invention also provides, in another aspect, a pharmaceutical composition (i.e., a pharmaceutical preparation) comprising an effective amount of a cadherin-11 antagonist according to the claims in a pharmaceutically acceptable carrier or formulated as an implant such as a stent. The invention also provides methods for making such compositions by placing the cadherin-11 antagonist in a pharmaceutically acceptable carrier. The pharmaceutical composition preparation may contain one or more cadherin-11 antagonists and, optionally, other therapeutic agents which are useful in the treatment of the particular disorder being treated.

These and other aspects of the invention, as well as various advantages and utilities, will be more apparent with reference to the detailed description of the preferred embodiments and to the accompanying drawings.

### BRIEF DESCRIPTION OF THE DRAWINGS

The Examples refer to and include a brief description of various figures. It is to be understood that the drawings or figures are illustrative only and are not required for the enablement of the inventions disclosed herein.

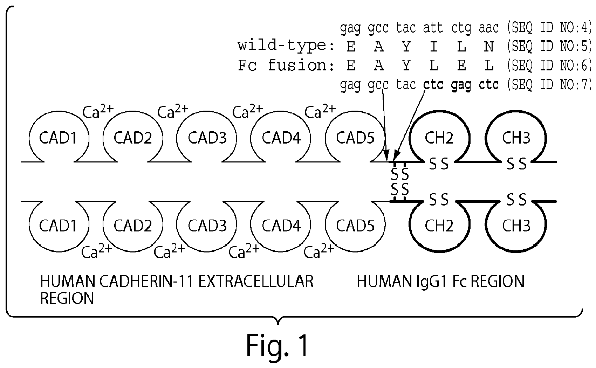

FIG. 1. A schematic of the structure of the human cadherin-11-Fc fusion protein. The sequence of the extracellular juxtamembrane region of wild-type cadherin-11 and the alterations resulting from fusion with the human Fc region are shown. Regions corresponding to the Fc portion are shown in bold.

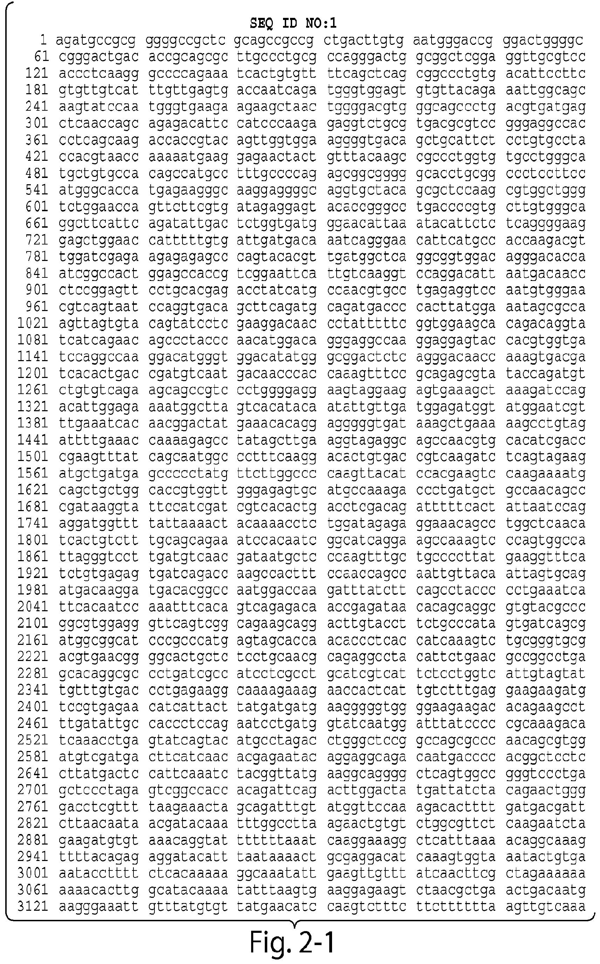

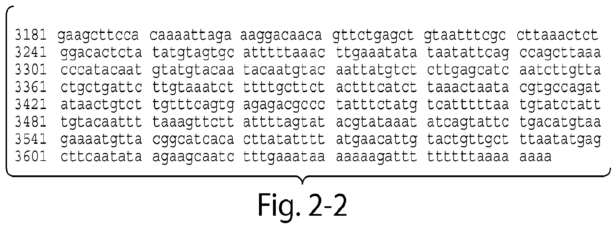

FIG. 2. Nucleotide sequence of human cadherin-11 (SEQ ID NO:1).

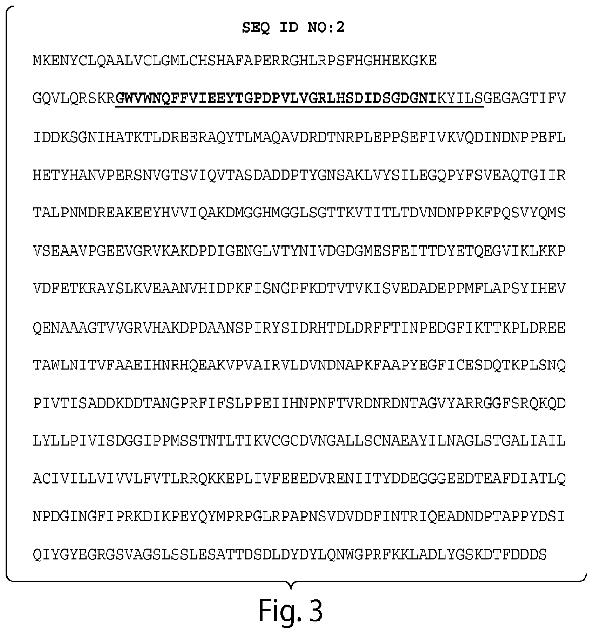

FIG. 3. Amino acid sequence of human cadherin-11 (SEQ ID NO:2).
FIG. 4. The design of anti-cadherin-11 monoclonal antibody (mAb) treatment of a mouse model of allergic skin inflammation.
FIG. 5. Anti-caderin-11 mAb treatment suppressed skin inflammation. (A) H & E staining of sections from SAL- and OVA-sensitized skin. (B) Epidermal and dermal thickness was significantly decreased by anti-cad-11 mAb treatment compared to the untreated group. (C) Eosinophils but not CD4⁺ T cells were significantly less recruited into the inflamed skin.
FIG. 6. Th2 type cytokines were down-regulated in sensitized skin of the anti-cad-11 mAb treated group. Total RNA isolated from sensitized skin was analyzed for (A) Th2 type cytokines (IL-4 and IL-13), IL-17, and IFN-γ, and (B) eotaxin-1, a chemokine for recruiting eosinophils.
FIG. 7. Cadherin-11-deficient mice are protected from colitis in the dextran sodium sulfate model of inflammatory bowel disease, as evidenced by effects on body weight (A), and colon length (B).

### DETAILED DESCRIPTION OF THE INVENTION

Cadherin-11 is a transmembrane molecule that, *inter alia,* mediates binding of cells to each other through interaction with itself or its counter-receptors. Like other cadherins, cadherin-11 is proposed to mediate, among other things, adhesion of like cells to each other as well as adhesion of cells of different lineages to each other. The human and mouse cadherin-11 genes have been isolated and sequenced previously (Suzuki S. et al. Cell Reg 2:261-70, 1991). See also, Genbank Accession No. NM_001797, (SEQ ID NO: 1 and SEQ ID NO: 2) for the human cadherin-11 cDNA and predicted amino acid sequences, respectively.

The invention is based at least in part on ability of cadherin-11 to modulate factor or cytokine secretion by fibroblasts in certain inflammatory disorders. The invention embraces the use of cadherin-11 antagonists in the treatment of certain inflammatory disorders that do not involve inflammation in the joints of subject.

The methods involve administering to a subject a cadherin-11 antagonist. Cadherin-11 antagonists are able to interfere with, including inhibit partially or completely (1) cell proliferation, (2) secretion of molecules such as, but not limited to, IL-6, MCP and TNF-alpha, (3) apoptosis, (4) migration and/or (5) attachment, of cadherin-11 expressing cells, and particularly cadherin-11 expressing fibroblasts that exist or are at least associated with inflammation. In important embodiments, such antagonists lead to a decrease in the production and/or secretion of molecules such as, but not limited to, cytokines such as IL-4, IL-6, IL-13, IL-15, IL-17, IL-23, type I IFNs, IL-1beta and TNF-alpha, growth factors such as GM-CSF, VEGF, TGF-beta, PDGF and SCF, chemokines such as CXCL-1, -5, -6, -8, -9, -10, -11, -12, -13, CCL-2, -3, -5, eotaxins such as eotaxin-1 (CCL11), and CX3CL1, bioactive lipids such as prostaglandins and leukotrienes, and degradative enzymes such as MMPs and cathepsins. In some instances, the antagonists inhibit or reduce a preexisting Th2 immune response. In some instances, the antagonists inhibit or reduce a preexisting Th17 immune response. In some instances, the antagonists inhibit or reduce Th2 and Th17 immune responses. In some instances, the antagonists reduce eosinophil numbers at a site of inflammation. The antagonists can also prevent or down-regulate the expression (or overexpression) of cell surface receptors on the fibroblast surface such as CD40, VCAM-1, ICAM-1, TLRs, integrins, and cytokine and chemokine receptors.

In one aspect, the disclosure is directed to a method for treating a subject having a non-joint inflammatory disorder. As used herein, a non-joint inflammatory disorder is an inflammatory disorder that does not occur or involve the joints. Inflammatory disorders are typically characterized by the presence of activated immune cells and/or increased levels of inflammatory markers such as a cytokine milieu that stimulates immune cells such as macrophages, neutrophils and other granulocytes, T cells and NK cells.

The inflammatory disorders to be treated according to the invention may be autoimmune disorders. An autoimmune disorder is one in which the body's immune system reacts against one or more body tissues and thus attacks the tissue as it would a foreign antigen or pathogen.

The inflammatory disorder to be treated according to the invention may be inflammatory bowel disease (e.g., colitis such as severe colitis, ulcerative colitis, Crohn's disease), psoriasis, Graves opthalmopathy, vasculitis (i.e., inflammation of the blood vessels), eczema such as atopic dermatitis, multiple sclerosis, atrial myxoma, inflammation resulting from solid organ transplantation (e.g., transplantation of heart, liver, kidney, skin, lung, eye, and the like), glomerulonephritis, interstitial nephritis, inflammation associated with a burn or other skin lesion, peritoneal inflammation and diverticulitis that occurs post-surgery, post-performation, or post-infection, or pleural inflammation that occurs post-surgery, post-trauma, or post-infection.

The inflammatory disorders to be treated by the methods described herein exclude acute and chronic synovitis, arthritis including psoriatic arthritis, rheumatoid arthritis, arthritis associated with ankylosing spondylitis, arthritis associated with systemic lupus erythrematosus, and juvenile chronic arthritis, chronic Lyme disease, and Reiter's syndrome.

The antagonist may be administered systemically or locally depending upon whether the disorder being treated is systemic or localized.

The cadherin-11 antagonist is administered to the subject in a therapeutically effective amount. A therapeutically effective amount is a dosage of the cadherin-11 antagonist sufficient to provide a medically desirable result. In the treatment methods of the disclosure the therapeutically effective amount of the cadherin-11 antagonist may be that amount which is sufficient to reduce inflammation (or the level of inflammatory markers such as but not limited to particular cell types such as eosinophils or particular cytokines or chemokines such as IL-4, IL-13, IL-17 or an eotaxin such as eotaxin-1) at a particular location or systemically (depending on the disorder), or to alleviate pain or other symptoms associated with the inflammatory disorder. As used herein, treatment embraces the use of the cadherin-11 antagonist in subjects that have the inflammatory disorder as well as in subjects at risk of developing the disorder.

In still other embodiments, the therapeutically effective amount is the amount that prevents or delays the onset of fibrosis in a subject. Fibrosis refers to the development of excess fibrous connective tissue in an organ or tissue. Fibrosis can occur in a variety of tissues or organs. Fibrotic conditions include lung fibrosis, hepatic fibrosis (e.g., associated with alcohol consumption, viral hepatitis, and/or schistosomiasis), hypertrophic scars, keloids, burns, Peyronie's disease, Dupuytren's contractures, myelofibrosis, pancreatic fibrosis, post-myocardial infarction cardiac fibrosis, kidney/renal fibrosis associated with diabetes, post-inflammatory renal fibrosis, and drug-induced fibrosis (e.g., resulting from chemotherapy and/or radiation exposure). In some embodiments, the subject does not have a fibrosis. In still other embodiments, the subject does not have fibrosis nor does it have an inflammatory joint disorder.

A subject, as used herein, refers to any mammal susceptible to having or presently having any of the afore-mentioned non-joint inflammatory disorder. The subjects may be human and non-human subjects. Non-human subjects include but are not limited to companion animals (e.g., dogs and cats), agricultural or competitive animals (e.g., cows, horses, etc.). Preferably, the subject is one having one of these non-joint inflammatory disorders. In other embodiments, the subject is at risk of developing fibrosis, and may or may not yet have an inflammatory disorder that precedes fibrosis. In certain embodiments, the subject does not have abnormal cadherin-11 mediated adhesion in the brain and/or liver.

### Cadherin-11 Antagonists:

As used herein, the term antagonist refers to any protein, polypeptide, peptide, peptidomimetic, glycoprotein, antibody, antibody fragment, carbohydrate, nucleic acid, organic molecule, inorganic molecule, large molecule, or small molecule that blocks, inhibits, reduces or neutralizes the function, activity and/or expression of another molecule. As used herein, a cadherin-11 antagonist is an agent that blocks, inhibits, reduces or neutralizes the function, activity and/or expression of cadherin-11. As described above, cadherin-11 is involved in cell attachment, interaction and/or migration. Cadherin-11 is known to bind to itself in what is referred to as homophilic or homotypic binding. The cadherin-11 antagonists may interfere with cadherin-11 homotypic binding or heterotypic binding (i.e., binding of cadherin-11 to a counter-receptor that is not cadherin-11). The cadherin-11 antagonist may interfere with cadherin-11 function by reducing the amount of cadherin-11 that is expressed by a cell or by interacting with cadherin-11 (or its counter-receptor) thereby preventing interaction of cadherin-11 with its target. Accordingly, the cadherin-11 antagonist may interfere, in whole or in part, with the transcription of cadherin-11 or with the translation of cadherin-11 (thereby interfering with cadherin-11 expression), or it may interfere with the ability of cadherin-11 to bind to another cadherin-11 or to another cadherin-11 counter-receptor. The cadherin-11 antagonist may reduce cadherin-11 function or activity by about 10%, 20%, 30%, 40%, 50%, 60%, 70%, 80%, 90% or 100%, relative to a control such as PBS. It will be understood that the cadherin-11 antagonist may be used in an amount that reduces cadherin-11 function or activity by about these amounts. It will further be understood that some cadherin-11 antagonists are preferably used in vitro while others are more suitable for the in vivo methods provided herein.

Some cadherin-11 antagonists bind to the extracellular domain of cadherin-11, some bind to particular regions of the extracellular domain of cadherin-11. As discussed herein, the cadherin-11 extracellular domain is comprised of five (5) subdomains each approximately about 110 amino acids in size. (See, for example, U.S. Patent No. 7589074 and Yagi et al. Genes and Development, 14:1169-1180, 2000.) The invention contemplates the use of cadherin-11 antagonists that bind to cadherin-11 EC1 or to a fragment of cadherin-11 EC1 (e.g., a fragment that comprises about the first 33 through to the first 37 amino acids of EC1), or to a fragment of cadherin-11 that comprises EC1 (or the first 33-37 amino acids of EC1). In some embodiments, the antagonist binds to a region of EC1 having an amino acid sequence of GWVWN QFFVI EEYTG PDPVL VGRLH SDIDS GDGN (SEQ ID NO:3, the first 34 amino acids of EC1). Alternatively or additionally, the antagonist may comprise some or all of this amino acid sequence.

The cadherin-11 antagonist may be a peptide or protein, or it may be a nucleic acid, or it may be a organic or inorganic small molecule. The antagonists may be naturally occurring or non-naturally occurring. They may be isolated from a naturally occurring source or they may be synthesized in vitro.

The cadherin-11 antagonists may be conjugated to another agent such as an imaging agent or a cytotoxic agent. Imaging agents may be used to visualize cadherin-11 expression in vitro (e.g., for immunohistochemical analysis) or in vivo (e.g., for body imaging). Examples include radionuclides, contrast agents, and particulates routinely used in medical imaging. Cytotoxic agents are agents that are toxic to cells. Examples include chemotherapeutic agents, toxins, and the like. The use of these agents conjugated to a cadherin-11 antagonist will target such agents to fibroblasts associated with inflammatory disorders. In these instances, therapeutic benefit may be provided by a combination of the cadherin-11 antagonist which interferes with the ability of cadherin-11 to secrete inflammatory factors such as but not limited to IL-6 and the cytotoxic agent which is directly toxic to the fibroblast.

### Cadherin-11 Binding Peptides:

Cadherin-11 antagonists that are peptide or protein in nature include (1) a full length cadherin-11 protein, (2) a fragment of the full length protein, wherein the fragment comprises the transmembrane domain of cadherin-11 or a fragment of the extracellular domain including for example a fragment comprising or consisting of EC1 (e.g., a fragment that comprises EC1, a fragment that comprises EC1 and EC2, a fragment that comprises EC1-EC3, a fragment that comprises EC1-EC4, a fragment that comprises EC1-EC5, a fragment that comprises EC1 and EC3, a fragment that comprises EC1 and EC4, a fragment that comprises EC1 and EC5), (3) a fragment of the full length protein, wherein the fragment comprises one or more of cadherin-11 extracellular subdomains (e.g., EC1, EC2, EC3, EC4, or EC5 of the 5 extracellular subdomains of cadherin-11, or any combination thereof), (4) fusion proteins that comprise full length cadherin-11 or a fragment thereof, and (5) antibodies and fragments thereof. In important embodiments, the cadherin-11 antagonist binds to and/or comprises the EC1 domain of cadherin-11 or a fragment thereof (such as SEQ ID NO:3 provided herein). Cadherin-11 antagonists that are peptide or protein in nature preferably will bind preferentially (or selectively) to cadherin-11. Preferential (or selective) binding to cadherin-11 means that the peptide or protein binds with greater affinity to cadherin-11 than to another protein. In some instances, the peptide or protein binds to cadherin-11 with an affinity that is about 2-fold more, about 3-fold more, about 4-fold more, about 5-fold more, about 10-fold more, about 25-fold more, about 50-fold more, about 100-fold more, about 1000-fold more, or more than its affinity for a protein that is not cadherin-11 or for any other moiety. Such differences in affinity are preferably manifest under physiological conditions as occur in vivo. In some embodiments, the cadherin-11 binding peptides bind to EC1 of cadherin-11, and optionally to the first 33-37 amino acids, including the first 33, first 34, first 35, first 36,or first 37 amino acids of EC1 of cadherin-11, as shown in SEQ ID NO:2 provided herein. Binding to this region of cadherin-11 can be determined through competitive binding assays using other binding agents known to bind to this region of cadherin-11 such as those described in WO2009/089062. The afore-mentioned antagonists are collectively referred to as cadherin-11 binding peptides. Cadherin-11 binding peptides may be harvested and isolated from naturally occurring sources or they may be synthesized and screened for their ability to bind to cadherin-11.

As used herein with respect to peptides and proteins, the term "isolated" means separated from its native environment in sufficiently pure form so that it can be manipulated or used for any one of the purposes of the invention.

Binding peptides can also be derived from sources other than antibody technology. For example, binding peptides can be provided by degenerate peptide libraries which can be readily prepared in solution, in immobilized form, as bacterial flagella peptide display libraries or as phage display libraries. Combinatorial libraries also can be synthesized of peptides containing one or more amino acids. Libraries can also be made that are comprised of peptides and non-peptide synthetic moieties.

Cadherin-11, or a fragment thereof, also can be used to isolate other cadherin-11 binding peptides or partners. Isolation of binding partners may be performed according to well-known methods. For example, cadherin-11 or a fragment thereof (e.g., an extracellular fragment) can be attached to a substrate, and then a putative cadherin-11 binding peptide may be applied to the substrate. If a cadherin-11 binding peptide is present, it will bind to the substrate-bound cadherin-11, and it can then be isolated and further analyzed.

### Full-Length Cadherin-11 and Cadherin-11 Fragments:

Based on the known nucleotide and amino acid sequence of cadherin-11, suitable fragments of cadherin-11 may be identified and generated using conventional technology. Reference may be made to U.S. Patent Nos. 5597725, 5639634, 5646250, 6787136, 6946768, 7488478, and 7589074, and PCT Patent Publication Nos. WO 93/21302 and WO2009/089062.

Examples of suitable fragments include those that consist of or comprise amino acids 1-40, 1-39, 1-38, 1-37, 1-36, 1-35, 1-34, 1-33, 1-32, 1-31 or 1-30 of cadherin EC1 or those that consist of or comprise amino acids 15-34, 15-35, 15-36, 15-37, 15-38, 15-39, or 15-40 of cadherin EC1. The first 40 amino acids of EC1 are underlined and the first 35 amino acids of EC1 are bolded in SEQ ID NO:2 as provided herein. Examples of suitable fragments are also provided in WO2009/089062 (represented by the amino acid sequences of SEQ ID NOs: 3, 10, 12, and 13, and also described in US 2009/0253200). Other fragments may comprise amino acids 1-160, or 1-259, or 1-269 of SEQ ID NO:2, and optionally they may lack amino acids 1-53 of SEQ ID NO:2 which represents the leader and pro-region of human cadherin-11.

Cadherin-11 binding peptides may also be variants of full-length cadherin-11 or cadherin-11 fragments. Such variants may differ from cadherin-11 amino acid sequence by a degree. For example, variants may be about 80%, about 85%, about 90%, about 91%, about 92%, about 93%, about 94%, about 95%, about 96%, about 97%, about 98%, or about 99% identical to full length cadherin-11 or to a cadherin-11 fragment. Variants may comprise a cadherin-11 fragment and additional flanking constituents at the amino and/or carboxy end of the fragment. Such constituents may be amino acid in nature. In all instances, the variants bind to cadherin-11 and interfere with cadherin-11 function or activity.

Cadherin-11 binding peptides may be at least 10, 20, 30, 40, 50, 60, 70, 80, 90, 100, 110, amino acids in length or longer. For example, they may be about or at least 220, 330, 440, 550 amino acids in length.

In some important embodiments, the cadherin-11 antagonist is a functionally equivalent peptide analog of cadherin-11. As used herein, the term functionally equivalent peptide analog refers to a peptide analog that is capable of inhibiting the binding of cadherin-11 to, for example, itself. Functionally equivalent peptide analogs of cadherin-11 are identified, for example, using in vitro adhesion assays that measure the ability of the peptide analog to inhibit cadherin-11-mediated adhesion either between cells expressing cadherin-11 or between isolated cadherin-11 proteins, or some combination thereof. Accordingly, exemplary functionally equivalent peptide analogs of cadherin-11 include analogs of full length cadherin-11 or a cadherin-11 fragment that for example comprises conservative amino acid substitutions relative to the wild-type sequence.

Still other cadherin-11 binding peptides are provided in PCT Published Application Nos. WO99/57149, WO2004/048411, and WO2009/089062.

### Cadherin-11 Fusion Proteins:

The cadherin-11 binding peptide can be a fusion protein. A fusion protein, as used herein, is a protein that contains peptide regions from at least two different proteins. For example, a cadherin-11 fusion protein contains amino acid sequence from cadherin-11 and at least one non-cadherin-11 protein. Such fusion proteins can be formed by fusing, usually at the nucleotide level, coding sequence from cadherin-11 to coding sequence from a non-cadherin-11 protein. Examples of cadherin-11 fusion proteins include cadherin-11 GST fusion protein, cadherin-11 Fc fusion protein, cadherin-11 beta-galactosidase fusion protein, cadherin-11 poly-His fusion protein, and cadherin-11 GFP fusion protein. Fc fusion proteins may comprise regions of the Ig constant domain, including without limitation the hinge region, the CH1 domain, the CH2 domain, and/or CH3 domain, optionally conjugated to the cadherin-11 fragment via the hinge domain. The Fc portion may derive from human antibodies or non-human antibodies. The antibodies may be IgG1 or IgG2, although they are not so limited. Methods of making Fc fusion proteins are known in the art and are described at least in EP0464533.

In some embodiments, the cadherin-11 fusion proteins comprise the entire extracellular domain of cadherin-11. In some embodiments, the cadherin-11 fusion protein comprises one or more extracellular subdomains of cadherin-11, such as EC1. Examples include fusion proteins comprising EC1, EC1/2, EC1-3, EC1-4, EC1/3, EC1/4, and EC1/5, or fragments of EC1. In important embodiments, the fusion protein binds to the EC1 domain of cadherin-11. Examples of cadherin-11 fusion proteins include cadherin-11-EC1-Fc fusion protein (comprising the EC1 domain of cadherin-11), cadherin-11-EC1/2-Fc fusion protein (comprising the EC1 and EC2 domains of cadherin-11), and cadherin-11-EC1-5-Fc fusion protein (comprising the EC1, EC2, EC3, EC4, and EC5 domains of cadherin-11). Some fusion proteins may comprise the first 40, first 39, first 38, first 37, first 36, first 35, or first 34 amino acids of the EC1 domain of cadherin-11, as described in WO 2009/089062.

Methods of synthesis of cadherin-11 fusion proteins can be found at least in U.S. Patent Nos. 5597725, 5639634, 5646250, 6787136, 6946768, 7488478, and 7589074 and PCT Patent Publication No. WO 93/21302 and WO2009/089062 (see for example SEQ ID NOs: 6 and 7, the nucleotide and amino acid sequences of a human cadherin-11-EC1-hIgG2-Fc fusion protein).

### Cadherin-11 Antibodies and Antibody Fragments:

Cadherin-11 antagonists that are cadherin-11 binding peptides may be antibodies or antigen-binding antibody fragments. The antibodies may be monoclonal antibodies or polyclonal antibodies. They may be chimeric antibodies including humanized antibodies. They may be four chain antibodies comprised of two heavy and two light chains, or they may be two chain antibodies such as those comprised of two heavy chains (such as camelid antibodies) or those comprised of a single heavy chain linked to a single light chain (such as a single chain Fvs). They can be of any type (e.g., IgG, IgE, IgM, IgD, IgA and IgY), class (e.g., IgG1, IgG2, IgG3, IgG4, IgA1 and IgA2) or subclass. As discussed below, these various antibody forms can be prepared according to conventional methodology. The antibodies and antibody fragments may be naturally occurring or non-naturally occurring including for example recombinantly produced antibodies and fragments.

Significantly, as is well-known in the art, only a small portion of an antibody molecule, the paratope, is involved in the binding of the antibody to its epitope (see, in general, Clark, W.R. (1986) The Experimental Foundations of Modern Immunology Wiley & Sons, Inc., New York; Roitt, I. (1991) Essential Immunology, 7th Ed., Blackwell Scientific Publications, Oxford). The pFc' and Fc regions, for example, are effectors of the complement cascade but are not involved in antigen binding. An antibody from which the pFc' region has been enzymatically cleaved, or which has been produced without the pFc' region, designated an F(ab')₂ fragment, retains both of the antigen binding sites of an intact antibody. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

The terms Fab, Fab', Fc, Fd, pFc', F(ab')₂, Fv, and dAb are employed with either standard immunological meanings [Klein, Immunology (John Wiley, New York, NY, 1982); Clark, W.R. (1986) The Experimental Foundations of Modern Immunology (Wiley & Sons, Inc., New York); Roitt, I. (1991) Essential Immunology, 7th Ed., (Blackwell Scientific Publications, Oxford)]. Well-known functionally active antibody fragments include but are not limited to F(ab')₂, Fab, Fv and Fd fragments of antibodies. These fragments which lack the Fc fragment of intact antibody, clear more rapidly from the circulation, and may have less non-specific tissue binding than an intact antibody (Wahl et al., J. Nucl. Med. 24:316-325 (1983)). For example, single-chain antibodies can be constructed in accordance with the methods described in U.S. Patent No. 4,946,778 to Ladner et al. Such single-chain antibodies include the variable regions of the light and heavy chains joined by a flexible linker moiety. Methods for obtaining a single domain antibody ("Fd") which comprises an isolated variable heavy chain single domain, also have been reported (see, for example, Ward et al., Nature 341:644-646 (1989), disclosing a method of screening to identify an antibody heavy chain variable region (V_{H} single domain antibody) with sufficient affinity for its target epitope to bind thereto in isolated form). Methods for making recombinant Fv fragments based on known antibody heavy chain and light chain variable region sequences are known in the art and have been described, e.g., Moore et al., US Patent No. 4,462,334. Other references describing the use and generation of antibody fragments include e.g., Fab fragments (Tijssen, Practice and Theory of Enzyme Immunoassays (Elsevieer, Amsterdam, 1985)), Fv fragments (Hochman et al., Biochemistry 12: 1130 (1973); Sharon et al., Biochemistry 15: 1591 (1976); Ehrilch et al., U.S. Patent No. 4,355,023) and portions of antibody molecules (Audilore-Hargreaves, U.S. patent No. 4,470,925). Thus, those skilled in the art may construct antibody fragments from various portions of intact antibodies without destroying the specificity of the antibodies.

Within the antigen-binding portion of an antibody, as is well-known in the art, there are complementarity determining regions (CDRs), which directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FR1 through FR4) separated respectively by three complementarity determining regions (CDR1 through CDR3). The CDRs, and in particular the CDR3 regions, and more particularly the heavy chain CDR3, are largely responsible for antibody specificity.

It is now well-established in the art that the non-CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody. Thus, for example, PCT International Publication No. WO 92/04381 and published European Patent EP 0239400 teach the production and use of humanized murine antibodies in which at least a portion of the murine FR regions have been replaced by FR regions of human origin. Such antibodies, including fragments of intact antibodies with antigen-binding ability, are often referred to as "chimeric" antibodies. There are entities in the United States which will synthesize humanized antibodies from specific murine antibody regions commercially, such as Protein Design Labs (Mountain View California), Abgenix, and Medarex.

Thus, as will be apparent to one of ordinary skill in the art, the present invention also provides for F(ab')₂, Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')₂ fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. The present invention also includes single chain antibodies.

In addition, human monoclonal antibodies may be made by any of the methods known in the art, such as those disclosed in US Patent No. 5,567,610, issued to Borrebaeck et al., US Patent No. 565,354, issued to Ostberg, US Patent No. 5,571,893, issued to Baker et al, Kozber, J. Immunol. 133: 3001 (1984), Brodeur, et al., Monoclonal Antibody Production Techniques and Applications, p. 51-63 (Marcel Dekker, Inc, new York, 1987), and Boerner et al., J. Immunol., 147: 86-95 (1991). In addition to the conventional methods for preparing human monoclonal antibodies, such antibodies may also be prepared by immunizing transgenic animals that are capable of producing human antibodies (e.g., Jakobovits et al., PNAS USA, 90: 2551 (1993), Jakobovits et al., Nature, 362: 255-258 (1993), Bruggermann et al., Year in Immunol., 7:33 (1993) and US Patent No. 5,569,825 issued to Lonberg).

Exemplary cadherin-11 antibodies and methods for making such antibodies are described in U.S. Patent Nos. 5597725, 5639634, 5646250, 6787136, 6946768, 7488478, and 7589074, and PCT Patent Publication No. WO 93/21302 and WO2009/089062. Examples of cadherin-11 antibodies include 23C6, 13C2, 27F3, 5F82 (commercially available from Lifespan Science), H1M1 antibody (cadherin-11 EC1 specific antibody produced by hybridoma H1M1 having ATCC Accession No. PTA-9699), H14 antibody (cadherin-11 EC1 specific antibody produced by hybridoma H14 having ATCC Accession No. PTA-9701), BM5096/1A6 (commercially available from Acris Antibodies GmbH), 283416 (commercially available from R&D Systems), and MAB2014 (commercially available from Millipore). Examples of cadherin-11 antibody fragments include the Fab fragment of antibodies 23C6, 13C2, 27F3, 5F82, H1M1 antibody, H14 antibody, BM5096/1A6, 283416, and MAB2014. The antibodies or antibody fragments may comprise one or more CDRs from known antibodies such as the H1M1 or H14 antibodies, as described in US 2009/0253200.

Antibodies and antibody fragments that bind to the EC1 domain of cadherin-11 are described in US 2009/0253200 and WO2009/089062.

Cadherin-11 antibodies may also be bispecific or bifunctional antibodies capable of binding to two different epitopes by virtue of their different antigen-binding sites.

Still other cadherin-11 antibodies are camelid antibodies as described in PCT Publication No. WO 94/04678 and U.S. Patent Publication No. 20080124324, and their derivatives in the form of camelid nanobodies as in U.S. Patent No. 5759808. Camelid antibodies and camelid nanobodies are commercially available from sources such as Ablynx (Belgium). It is to be understood that the cadherin-11 camelid antibodies can be humanized in a manner similar to that described herein for other antibody types.

In some embodiments, the antagonists do not include the antibodies disclosed in U.S. Patent 5,597,725 and in PCT application PCT/US93/03681 (WO/93/21302). Thus, in certain related embodiments, the agents of the disclosure do not embrace the monoclonal antibodies produced by the hybridomas designated 30Q8A (HB11316), 30Q4H (HB11317), 45A5G (HB 11318), 30S2F (HB11319), 45C6A (HB 11320), 30T11G (HB 11324), 64G11F (HB 11527).

### Cadherin-11 Nucleic Acid Antagonists:

A cadherin-11 antagonist may also be a nucleic acid. These antagonists include nucleic acids that (1) encode a cadherin-11 polypeptide or a fragment thereof; (2) are cadherin-11 antisense molecules which inhibit the transcription or translation of the foregoing nucleic acid molecules; (3) are cadherin-11 inhibitory RNA (RNAi or siRNA); (4) are cadherin-11 ribozymes; (5) aptamers that are nucleic acid in nature but bind to the cadherin-11 as would binding peptides thereby interfering with the binding of cadherin-11 to another cadherin-11 or to another cadherin-11 counter-receptor. In some embodiments, a cadherin-11 antagonist that is a nucleic acid (1) hybridizes under stringent conditions to a nucleic acid having a sequence of SEQ ID NO: 1, and (2) codes for a cadherin-11 polypeptide or a fragment thereof that is capable of binding specifically to cadherin-11.

### Cadherin-11 Encoding Nucleic Acids:

Cadherin-11 antagonists include nucleic acids that encode cadherin-11 and fragments of cadherin-11. The cadherin-11 full length nucleotide sequence is provided as SEQ ID NO:1. Nucleic acids comprising a nucleotide sequence of SEQ ID NO:1 may be used as antagonists, as an example. The cadherin-11 antagonists of the invention also include homologs and alleles of a nucleic acid molecule comprising a sequence of SEQ ID NO: 1.

The cadherin-11 nucleic acid antagonists, may encode polypeptides which are soluble cadherin-11 polypeptides, membrane-bound polypeptides, or cadherin-11 fragments such as fragments that consist of or comprise EC1 or a fragment thereof (e.g., the first 33-37 amino acids of EC1). The soluble cadherin-11 polypeptides lack a transmembrane domain and, optimally, contain further amino acids which render the polypeptide soluble (e.g., fusion proteins, containing all or part of cadherin-11, which inhibit the binding of cadherin-11 to another cadherin-11). Cadherin-11 fragments which are membrane-bound (or membrane associated) preferably contain a transmembrane domain. Cadherin-11 nucleic acid antagonists further embrace nucleic acid molecules which code for a cadherin-11 protein having the amino acid sequence of SEQ ID NO: 2 (or SEQ ID NO:3, for example), but which may differ from the sequence of SEQ ID NO: 1 due to the degeneracy of the genetic code.

Certain cadherin-11 nucleic acid antagonists can be identified by conventional techniques, e.g., by identifying nucleic acid sequences which code for cadherin-11 and which hybridize to a nucleic acid molecule having the sequence of SEQ ID NO: 1 under stringent conditions. The term "stringent conditions," as used herein, refers to parameters with which the art is familiar. More specifically, stringent conditions, as used herein, refer to hybridization at 65C in hybridization buffer (3.5 x SSC, 0.02 % formamide, 0.02 % polyvinyl pyrolidone, 0.02 % bovine serum albumin, 2.5 mM NaH₂PO₄ (pH 7), 0.5 % SDS, 2 mM EDTA). SSC is 0.15 M sodium chloride/0.15 M sodium citrate, pH 7; SDS is sodium dodecyl sulphate; and EDTA is ethylenediaminetetraacetic acid. After hybridization, the membrane to which the DNA is transferred is washed at 2x SSC at room temperature and then at 0.1x SSC/0.1x SDS at 65C.

There are other conditions, reagents, and so forth which can be used, which result in a similar degree of stringency. The skilled artisan will be familiar with such conditions and, thus, they are not given here. It will be understood, however, that the skilled artisan will be able to manipulate the conditions in a manner to permit the clear identification of homologs and alleles of the nucleic acid molecules of the invention. The skilled artisan also is familiar with the methodology for screening cells and libraries for the expression of further nucleic acids molecules which can be isolated and sequenced. In screening for cadherin-11 sequences for example, a Southern blot may be performed using the foregoing conditions, together with a radioactive probe. After washing the membrane to which the DNA is finally transferred, the membrane can be placed against x-ray film to detect the radioactive signal.

In general, cadherin-11 homologs and alleles typically will share at least 70% nucleotide identity with SEQ. ID. NO: 1; and in some instances, will share at least 75% nucleotide identity; and in still other instances, will share at least 80% nucleotide identity. Watson-Crick complements of the foregoing nucleic acids are also embraced by the invention. The preferred cadherin-11 homologs have at least 85% sequence homology to SEQ. ID. NO: 1. More preferably the cadherin-11 homologs have at least 90% and most preferably at least 95% sequence homology to SEQ. ID. NO: 1. The homology can be calculated using various, publicly available software tools developed by NCBI (Bethesda, Maryland) that can be obtained through the internet. Exemplary tools include the BLAST system available at the NCBI website. Pairwise and ClustalW alignments (BLOSUM30 matrix setting) as well as Kyte-Doolittle hydropathic analysis can be obtained using the MacVector sequence analysis software (Oxford Molecular Group).

The disclosure also includes degenerate nucleic acids which include alternative codons to those present in the naturally occurring nucleic acid that encodes, for example, the human cadherin-11 polypeptide. As is well known in the art, and as an example, serine residues are encoded by the codons TCA, AGT, TCC, TCG, TCT and AGC. Each of the six codons is equivalent for the purposes of encoding a serine residue. Thus, it will be apparent to one of ordinary skill in the art that any of the serine-encoding nucleotide codons may be employed to direct the protein synthesis apparatus, in vitro or in vivo, to incorporate a serine residue. Similarly, nucleotide sequence triplets which encode other amino acid residues include, but are not limited to, CCA, CCC, CCG and CCT (proline codons); CGA, CGC, CGG, CGT, AGA and AGG (arginine codons); ACA, ACC, ACG and ACT (threonine codons); AAC and AAT (asparagine codons); and ATA, ATC and ATT (isoleucine codons). Other amino acid residues may be encoded similarly by multiple nucleotide sequences.

As used herein with respect to nucleic acids, the term "isolated" means: (i) amplified *in vitro* by, for example, polymerase chain reaction (PCR); (ii) recombinantly produced by cloning; (iii) purified, as by cleavage and gel separation; or (iv) synthesized by, for example, chemical synthesis. An isolated nucleic acid is one which is readily manipulable by recombinant DNA techniques well known in the art. Thus, a nucleotide sequence contained in a vector in which 5' and 3' restriction sites are known or for which polymerase chain reaction (PCR) primer sequences have been disclosed is considered isolated but a nucleic acid sequence existing in its native state in its natural host is not. An isolated nucleic acid may be substantially purified, but need not be. For example, a nucleic acid that is isolated within a cloning or expression vector is not pure in that it may comprise only a tiny percentage of the material in the cell in which it resides. Such a nucleic acid is isolated, however, as the term is used herein because it is readily manipulable by standard techniques known to those of ordinary skill in the art.

The cadherin-11 nucleic acid antagonist, in one embodiment, is operably linked to a gene expression sequence which directs the expression of the cadherin-11 nucleic acid antagonist within a cell such as a eukaryotic cell. The "gene expression sequence" is any regulatory nucleotide sequence, such as a promoter sequence or promoter-enhancer combination which facilitates the efficient transcription and translation of the cadherin-11 nucleic acid antagonist to which it is operably linked. The gene expression sequence may, for example, be a mammalian or viral promoter, such as a constitutive or inducible promoter. Constitutive mammalian promoters include, but are not limited to, the promoters for the following genes: hypoxanthine phosphoribosyl transferase (HPTR), adenosine deaminase, pyruvate kinase, beta-actin promoter and other constitutive promoters. Exemplary viral promoters which function constitutively in eukaryotic cells include, for example, promoters from the simian virus, papilloma virus, adenovirus, human immunodeficiency virus (HIV), Rous sarcoma virus, cytomegalovirus, the long terminal repeats (LTR) of moloney leukemia virus and other retroviruses, and the thymidine kinase promoter of herpes simplex virus. Other constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art.

In general, the gene expression sequence shall include, as necessary, 5' non-transcribing and 5' non-translating sequences involved with the initiation of transcription and translation, respectively, such as a TATA box, capping sequence, CAAT sequence, and the like. Especially, such 5' non-transcribing sequences will include a promoter region which includes a promoter sequence for transcriptional control of the operably joined cadherin-11 nucleic acid antagonist. The gene expression sequences optionally includes enhancer sequences or upstream activator sequences as desired.

Cadherin-11 nucleic acid antagonist may be used in both in vivo and in vitro methods. Nucleic acid molecules of the invention may be introduced into a cell in vitro, followed by the transfer of the cell to the site of fibrosis. The cell into which the nucleic acid molecule is introduced may be harvested from the site of fibrosis (e.g., a fibroblast) or it may be a cell which is not normally present at the site of inflammation. A sequence which permits expression of the nucleic acid in a particular tissue (or cell), such as for example the lung, is one which is selectively transcriptionally active in the tissue (or cell) and thereby causes the expression of the nucleic acid in the tissue (or cell). Those of ordinary skill in the art will be able to easily identify alternative promoters that are capable of expressing such a nucleic acid molecule in lung tissue, liver tissue, renal tissue, and the like, as mentioned herein. Alternatively, a cell transduced with the cadherin-11 nucleic acid antagonist may be cultured in vitro in order to produce a cadherin-11 protein antagonist or it may be used in in vitro screening assays. For example, the gene expression sequence may be used to express cadherin-11 in a cell which does not inherently express cadherin-11.

The nucleic acid molecule sequences of the invention and the gene expression sequence are said to be "operably linked" when they are covalently linked in such a way as to place the transcription and/or translation of the nucleic acid antagonist (e.g., a cadherin-11 coding sequence) under the influence or control of the gene expression sequence. If it is desired that nucleic acid molecule be translated into a functional protein, two DNA sequences are said to be operably linked if induction of a promoter in the 5' gene expression sequence results in the transcription of the nucleic acid molecule and if the nature of the linkage between the two DNA sequences does not (1) result in the introduction of a frame-shift mutation, (2) interfere with the ability of the promoter region to direct the transcription of the nucleic acid molecule, or (3) interfere with the ability of the corresponding RNA transcript to be translated into a polypeptide. Thus, a gene expression sequence would be operably linked to a nucleic acid molecule if the gene expression sequence were capable of effecting transcription of that nucleic acid molecule such that the resulting transcript might be translated into the desired polypeptide.

### Cadherin-11 siRNA:

The invention contemplates the use of RNA interference agents such as siRNA and shRNA as cadherin-11 antagonists. siRNA are RNA molecules capable of causing interference and thus post-transcriptional silencing of specific genes in cells, including mammalian cells. siRNA comprise a double stranded region that is typically about 5-50 base pairs, more typically 10-40 base pairs, and even more typically 15-30 base pairs in length. The siRNA may be 20-50, 25-50 or 30-40 base pairs in length. These siRNA may be digested by the RNase III Dicer to yield smaller siRNA in the range of 19-28 base pairs, including 19 base pairs, 21 base pairs, 23 base pairs, 25 base pairs, and 27 base pairs in length. It is known that siRNA in this size range can be incorporated into and acted upon by the enzyme complex called RNA-Induced Silencing Complex (RISC), with a net result of target RNA degradation and/or inhibition of any protein translation therefrom. In a similar manner, double-stranded RNAs with other regulatory functions such as microRNAs (miRNA) can also be used. Reference can be made to Bass, Nature 411: 428-29 (2001); Elbashir et al., Nature 411: 494-98 (2001); Fire et al., Nature 391: 806-11 (1998); WO 01/75164, and US Patents 6506559, 7056704, 7078196, 7432250, for greater detail on siRNA as well as methods of making siRNA. siRNA to cadherin-11 are commercially available from sources such as Dharmacon.

siRNA forms such as the R- and L-form will have overhangs on one or both ends. As discussed herein, an R-form siRNA has a 3' overhang on its antisense strand. It may be blunted on its other end and/or it may have a 3' overhang on its other end, including an overhang comprising DNA residues. Alternatively, an L-form siRNA has a 3' overhang on its sense strand. It may be blunted on its other end and/or it may have a 3' overhang on its other end, including an overhang comprising DNA residues.

siRNA may be comprised of ribonucleotides or a combination of ribonucleotides and deoxyribonucleotides, including in some instances modified versions of one or both. For example, ribonucleotides containing a non-naturally occurring base (instead of a naturally occurring base) such as uridines and/or cytidines modified at the 5-position, e.g. 5-(2-amino)propyl uridine, 5-bromo uridine, or adenosines and/or guanosines modified at the 8-position, e.g. 8-bromo guanosine, or deaza nucleotides, e.g. 7-deaza-adenosine, or O- and N-alkylated nucleotides, e.g. N6-methyl adenosine can be incorporated into the siRNA. As another example, sugar-modified ribonucleotides having a 2' OH-group replaced by a group selected from H, OR, R, halo, SH, SR, NH₂, NHR, NR₂ or CN, wherein R is C₁-C₆ alkyl, alkenyl or alkynyl and halo is F, Cl, Br or I. As yet another example, the backbone may be modified to comprise modified backbone linkages such as but not limited to phosphorothioates. The siRNA may comprise modifications at the base, sugar and/or backbone, including a variety of such modifications.

Thus, siRNA molecules can be provided as and/or derived from one or more forms including, e.g., as one or more isolated small-interfering RNA (siRNA) double stranded duplexes, as longer double-stranded RNA (dsRNA), or as siRNA or dsRNA transcribed from a transcriptional cassette in a DNA plasmid. The siRNA molecules may have overhangs (e.g., 3' or 5' overhangs as described in Elbashir et al., Genes Dev., 15:188 (2001) or Nykanen et al., Cell, 107:309 (2001)), or may lack overhangs (i.e., have blunt ends). The person of ordinary skill in the art will appreciate and understand how such starting sources may be modified in order to arrive at the R- and L-forms described herein.

siRNA are targeted to genes in vivo or in vitro if all or part of the nucleotide sequence of their duplex (or double stranded) is complementary to a nucleotide sequence of the targeted gene, such as cadherin-11. siRNA made be synthesized based upon known (or predicted) nucleotide sequences of nucleic acids that encode proteins or other gene products. The sequence may be complementary to a translated or untranslated sequence in the target. The degree of complementarity between the siRNA and the target may be 100% or less than 100%, provided that sufficient identity exists to a target to mediate target-specific silencing. The art is familiar with efficacious siRNA that are less than 100% complementary to their target.

The level of silencing or interference may be measured in any number of ways, including quantitation of mRNA species and/or protein species. In some instances, mRNA quantitation is preferred particularly where the protein is intracellular or otherwise difficult to observe and/or assay. mRNA levels may be measured using RT-PCR or RACE, as an example. Protein levels may be measured using immunohistochemical staining. mRNA or protein levels may be reduced by 10%, 20%, 30%, 40%, 50%, 60%, 70%, 80%, 90%, 95%, 99%, or even 100%. Depending on the application, partial reduction (i.e., less than 100% may be sufficient) as compared to the level in the absence of the exogenously applied siRNA. In some embodiments, the level is reduced by 80% or more than 80% as compared to a control that has not been exposed to exogenously applied siRNA.

### Cadherin-11 Ribozymes:

A cadherin-11 ribozyme is an enzymatic RNA molecule capable of catalyzing the specific cleavage of cadherin-11 RNA. The cadherin-11 ribozyme binds to cadherin-11 RNA in a sequence specific manner (i.e., via sequence specific hybridization), and this is followed by endonucleolytic cleavage of the cadherin-11 RNA. Examples of ribozymes include engineered hairpin or hammerhead motif ribozymes. Ribozyme sequences complementary to a target such as cadherin-11 can be identified by scanning the target for ribozyme cleavage sites (e.g., GUA, GUU, and GUC), and then generating a sequence having about 15-20 ribonucleotides spanning the cleavage site.

### Cadherin-11 Antisense:

The cadherin-11 nucleic acid antagonist may be an antisense molecule (or oligonucleotide). Antisense oligonucleotides that selectively bind to a nucleic acid molecule encoding a cadherin-11 polypeptide, or a fragment thereof, to decrease cadherin-11 activity or function are embraced by the present invention. As used herein, the term "antisense oligonucleotide" or "antisense" describes an oligonucleotide that is an oligoribonucleotide, oligodeoxyribonucleotide, modified oligoribonucleotide, or modified oligodeoxyribonucleotide which hybridizes under physiological conditions to DNA comprising a particular gene or to an mRNA transcript of that gene and, thereby, inhibits the transcription of that gene and/or the translation of that mRNA. The antisense molecules are designed so as to interfere with transcription or translation of a target gene upon hybridization with the target gene or transcript. Those skilled in the art will recognize that the exact length of the antisense oligonucleotide and its degree of complementarity with its target will depend upon the specific target selected, including the sequence of the target and the particular bases which comprise that sequence. It is preferred that the antisense oligonucleotide be constructed and arranged so as to bind selectively with the target under physiological conditions, i.e., to hybridize substantially more to the target sequence than to any other sequence in the target cell under physiological conditions. Based upon SEQ ID NO:1 or upon allelic or homologous genomic and/or cDNA sequences, one of skill in the art can easily choose and synthesize any of a number of appropriate antisense molecules for use in accordance with the present invention. In order to be sufficiently selective and potent for inhibition, such antisense oligonucleotides should comprise at least 10 and, more preferably, at least 15 consecutive bases which are complementary to the target, although in certain cases modified oligonucleotides as short as 7 bases in length have been used successfully as antisense oligonucleotides (Wagner et al., Nat. Med. 1(11):1116-1118, 1995). Most preferably, the antisense oligonucleotides comprise a complementary sequence of 20-30 bases.

Although oligonucleotides may be chosen which are antisense to any region of the gene or mRNA transcripts, in preferred embodiments the antisense oligonucleotides correspond to N-terminal or 5' upstream sites such as translation initiation, transcription initiation or promoter sites. In addition, 3'-untranslated regions may be targeted by antisense oligonucleotides. Targeting to mRNA splicing sites has also been used in the art but may be less preferred if alternative mRNA splicing occurs. In addition, the antisense is targeted, preferably, to sites in which mRNA secondary structure is not expected (see, e.g., Sainio et al., Cell Mol. Neurobiol. 14(5):439-457, 1994) and at which proteins are not expected to bind. Finally, although SEQ ID NO:1 discloses a cDNA sequence, one of ordinary skill in the art may easily derive the genomic DNA corresponding to this sequence. Thus, the present invention also provides for antisense oligonucleotides which are complementary to the genomic DNA corresponding to SEQ ID NO:1. Similarly, antisense to allelic or homologous cadherin-11 or alternatively, cadherin-11 counter-receptor cDNAs and genomic DNAs are enabled without undue experimentation.

In one set of embodiments, the antisense oligonucleotides of the invention may be composed of "natural" deoxyribonucleotides, ribonucleotides, or any combination thereof. That is, the 5' end of one native nucleotide and the 3' end of another native nucleotide may be covalently linked, as in natural systems, via a phosphodiester internucleoside linkage. These oligonucleotides may be prepared by art recognized methods which may be carried out manually or by an automated synthesizer. They also may be produced recombinantly by vectors.

In preferred embodiments, however, the antisense oligonucleotides of the invention also may include "modified" oligonucleotides. That is, the oligonucleotides may be modified in a number of ways which do not prevent them from hybridizing to their target but which enhance their stability or targeting or which otherwise enhance their therapeutic effectiveness.

The term "modified oligonucleotide" as used herein describes an oligonucleotide in which (1) at least two of its nucleotides are covalently linked via a synthetic internucleoside linkage (i.e., a linkage other than a phosphodiester linkage between the 5' end of one nucleotide and the 3' end of another nucleotide) and/or (2) a chemical group not normally associated with nucleic acids has been covalently attached to the oligonucleotide. Preferred synthetic internucleoside linkages are phosphorothioates, alkylphosphonates, phosphorodithioates, phosphate esters, alkylphosphonothioates, phosphoramidates, carbamates, carbonates, phosphate triesters, acetamidates, carboxymethyl esters and peptides.

The term "modified oligonucleotide" also encompasses oligonucleotides with a covalently modified base and/or sugar. For example, modified oligonucleotides include oligonucleotides having backbone sugars which are covalently attached to low molecular weight organic groups other than a hydroxyl group at the 3' position and other than a phosphate group at the 5' position. Thus modified oligonucleotides may include a 2'-O-alkylated ribose group. In addition, modified oligonucleotides may include sugars such as arabinose instead of ribose.

As used herein with respect to polypeptides, the term "isolated" means separated from its native environment in sufficiently pure form so that it can be manipulated or used for any one of the purposes of the invention. Thus, isolated means sufficiently pure to be used (i) to raise and/or isolate antibodies, (ii) as a reagent in an assay, or (iii) for sequencing, etc.

As used herein with respect to nucleic acids, the term "isolated" means: (i) amplified *in vitro* by, for example, polymerase chain reaction (PCR); (ii) recombinantly produced by cloning; (iii) purified, as by cleavage and gel separation; or (iv) synthesized by, for example, chemical synthesis. An isolated nucleic acid is one which is readily manipulable by recombinant DNA techniques well known in the art. Thus, a nucleotide sequence contained in a vector in which 5' and 3' restriction sites are known or for which polymerase chain reaction (PCR) primer sequences have been disclosed is considered isolated but a nucleic acid sequence existing in its native state in its natural host is not. An isolated nucleic acid may be substantially purified, but need not be. For example, a nucleic acid that is isolated within a cloning or expression vector is not pure in that it may comprise only a tiny percentage of the material in the cell in which it resides. Such a nucleic acid is isolated, however, as the term is used herein because it is readily manipulable by standard techniques known to those of ordinary skill in the art.

In general, the antagonists of the invention are divided into two classes: biological vectors and chemical/physical vectors. Biological vectors are useful for delivery/uptake of nucleic acids to/by a target cell. Biological vectors include, but are not limited to, plasmids, phagemids, viruses, other vehicles derived from viral or bacterial sources that have been manipulated by the insertion or incorporation of the nucleic acid sequences of the invention, and additional nucleic acid fragments (e.g., enhancers, promoters) which can be attached to the nucleic acid sequences of the invention. Viral vectors are a preferred type of biological vector and include, but are not limited to, nucleic acid sequences from the following viruses: adenovirus; adeno-associated virus; retrovirus, such as moloney murine leukemia virus; harvey murine sarcoma virus; murine mammary tumor virus; rouse sarcoma virus; SV40-type viruses; polyoma viruses; Epstein-Barr viruses; papilloma viruses; herpes virus; vaccinia virus; polio virus; and RNA virus such as a retrovirus. One can readily employ other vectors not named but known in the art.

In addition to the biological vectors, chemical/physical vectors are useful for delivery/uptake of nucleic acids or polypeptides to/by a target cell. As used herein, a "chemical/physical vector" refers to a natural or synthetic molecule, other than those derived from bacteriological or viral sources, capable of delivering the cadherin-11 antagonist to a cell.

A preferred chemical/physical vector of the invention is a colloidal dispersion system. Colloidal dispersion systems include lipid-based systems including oil-in-water emulsions, micelles, mixed micelles, and liposomes. A preferred colloidal system of the invention is a liposome. Liposomes are artificial membrane vessels which are useful as a delivery vector in vivo or in vitro. It has been shown that large unilamellar vessels (LUV), which range in size from 0.2 - 4.0 µM can encapsulate large macromolecules. RNA, DNA, and intact virions can be encapsulated within the aqueous interior and be delivered to cells in a biologically active form (Fraley, et al., Trends Biochem. Sci., v. 6, p. 77 (1981)). In order for a liposome to be an efficient gene transfer vector, one or more of the following characteristics should be present: (1) encapsulation of the gene of interest at high efficiency with retention of biological activity; (2) preferential and substantial binding to a target cell in comparison to non-target cells; (3) delivery of the aqueous contents of the vesicle to the target cell cytoplasm at high efficiency; and (4) accurate and effective expression of genetic information.

Liposomes may be targeted to a particular tissue, by coupling the liposome to a specific ligand such as a monoclonal antibody, sugar, glycolipid, or protein specific for the particular tissue or cell type. Additionally, the vector may be coupled to a nuclear targeting peptide, which will direct the cadherin-11 modulating nucleic acid molecule to the nucleus of the host cell.

Liposomes are commercially available from Gibco BRL, for example, as LIPOFECTIN™ and LIPOFECTACE™, which are formed of cationic lipids such as N-[1-(2, 3 dioleyloxy)-propyl]-N, N, N-trimethylammonium chloride (DOTMA) and dimethyl dioctadecylammonium bromide (DDAB). Methods for making liposomes are well known in the art and have been described in many publications. Liposomes also have been reviewed by Gregoriadis, G. in Trends in Biotechnology, V. 3, p. 235-241 (1985).

The disclosure contemplates administration of a cadherin-11 antagonist to a subject as well as ex vivo contact of cells from the subject, particularly fibroblasts associated with the inflammatory disorder, with the cadherin-11 antagonist ex vivo, followed by re-introduction of the antagonist into the subject.

The invention further provides a pharmaceutical composition (i.e., a pharmaceutical preparation) for modulating a cadherin-11 function in a subject. The composition includes a pharmaceutically acceptable carrier and a cadherin-11 antagonist as defined in the claims.

The pharmaceutical preparations, as described above, are administered in effective amounts. For therapeutic applications, it is generally that amount sufficient to achieve a medically desirable result. In general, a therapeutically effective amount is that amount necessary to delay the onset of, inhibit the progression of, or halt altogether the particular condition being treated. As an example, the effective amount is generally that amount which serves to alleviate the symptoms (e.g., pain, inflammation, etc.) of the disorders described herein. The effective amount will depend upon the mode of administration, the particular condition being treated and the desired outcome. It will also depend upon the stage of the condition, the severity of the condition, the age and physical condition of the subject being treated, the nature of concurrent therapy, if any, the duration of the treatment, the specific route of administration and like factors within the knowledge and expertise of the medical practitioner. For prophylactic applications, it is that amount sufficient to delay the onset of, inhibit the progression of, or halt altogether the particular condition being prevented, and may be measured by the amount required to prevent the onset of symptoms.

Generally, doses of active compounds of the present invention would be from about 0.01 mg/kg per day to 1000 mg/kg per day, preferably from about 0.1 mg/kg to 200 mg/kg and most preferably from about 0.2 mg/kg to about 20 mg/kg, in one or more dose administrations daily, for one or more days. It is expected that doses ranging from 1-500 mg/kg, and preferably doses ranging from 1-100 mg/kg, and even more preferably doses ranging from 1-50 mg/kg, will be suitable. The preferred amount can be determined by one of ordinary skill in the art in accordance with standard practice for determining optimum dosage levels of the agent. It is generally preferred that a maximum dose of a cadherin-11 antagonist that is the highest safe dose according to sound medical judgment be used.

The cadherin-11 antagonists of the invention can be administered to a subject in need of such treatment in combination with concurrent therapy for treating an inflammatory disorder. The concurrent therapy may be invasive, or may involve drug therapy. The drug therapies are administered in amounts which are effective to achieve the physiological goals (e.g., to reduce inflammation), in combination with, for example, the cadherin-11 antagonist of the invention. Thus, it is contemplated that the drug therapies may be administered in amounts which are not capable of preventing or reducing the physiological consequences of an inflammatory disorder when the drug therapies are administered alone but which are capable of reducing the consequences when administered in combination with the cadherin-11 antagonist of the invention.

The cadherin-11 antagonist may be administered alone or in combination with the above-described drug therapies as part of a pharmaceutical composition. Such a pharmaceutical composition may include the cadherin-11 antagonist in combination with any standard physiologically and/or pharmaceutically acceptable carriers which are known in the art. The compositions should be sterile and contain a therapeutically effective amount of the cadherin-11 antagonist in a unit of weight or volume suitable for administration to a patient.

The term "pharmaceutically-acceptable carrier" as used herein means one or more compatible solid or liquid filler, diluents or encapsulating substances which are suitable for administration into a human or other animal. The term "pharmaceutically acceptable" means a non-toxic material that does not interfere with the effectiveness of the biological activity of the active ingredients. Pharmaceutically acceptable further means a non-toxic material that is compatible with a biological system such as a cell, cell culture, tissue, or organism. The term "carrier" denotes an organic or inorganic ingredient, natural or synthetic, with which the active ingredient is combined to facilitate the application. The characteristics of the carrier will depend on the route of administration. The components of the pharmaceutical compositions also are capable of being commingled with the agents of the present invention, and with each other, in a manner such that there is no interaction which would substantially impair the desired pharmaceutical efficacy. The pharmaceutically acceptable carrier must be sterile for in vivo administration. Physiologically and pharmaceutically acceptable carriers include diluents, fillers, salts, buffers, stabilizers, solubilizers, and other materials which are well known in the art.

Compositions suitable for parenteral administration conveniently comprise a sterile aqueous preparation of the cadherin-11 antagonists, which is preferably isotonic with the blood of the recipient. This aqueous preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose, any bland fixed oil may be employed including synthetic mono- or di-glycerides. In addition, fatty acids such as oleic acid may be used in the preparation of injectables. Carrier formulations suitable for oral, subcutaneous, intravenous, intramuscular, etc. administrations can be found in Remington's Pharmaceutical Sciences, Mack Publishing Co., Easton, PA.

A variety of administration routes are available. The particular mode selected will depend, of course, upon the particular drug selected, the severity of the condition being treated, and the dosage required for therapeutic efficacy. The methods of the invention, generally speaking, may be practiced using any mode of administration that is medically acceptable, meaning any mode that produces effective levels of the active compounds without causing clinically unacceptable adverse effects. Such modes of administration include oral, rectal, topical, nasal, interdermal, or parenteral routes. The term "parenteral" includes subcutaneous, intravenous, intramuscular, or infusion. Intravenous or intramuscular routes are not particularly suitable for long-term therapy and prophylaxis. They could, however, be preferred in emergency situations. Oral administration will be preferred for prophylactic treatment because of the convenience to the patient as well as the dosing schedule. It will be understood that the route of administration may also depend in some instances on the condition being treated. For example, if the condition is topical (e.g., atopic dermatitis or eczema), then the antagonists may be applied topically, intradermally or subcutaneously. Topical administration may be achieved through the use of pads, gauzes, bandages, compression garments, creams, lotions, sprays, emollients, and the like, all of which comprise the antagonist of interest.

The pharmaceutical compositions may conveniently be presented in unit dosage form and may be prepared by any of the methods well-known in the art of pharmacy. All methods include the step of bringing the cadherin-11 antagonists into association with a carrier which constitutes one or more accessory ingredients. In general, the compositions are prepared by uniformly and intimately bringing the cadherin-11 antagonists into association with a liquid carrier, a finely divided solid carrier, or both, and then, if necessary, shaping the product. Compositions suitable for oral administration may be presented as discrete units, such as capsules, tablets, lozenges, each containing a predetermined amount of the cadherin-11 antagonists. Other compositions include suspensions in aqueous liquids or non-aqueous liquids such as a syrup, elixir or an emulsion.

In one particular embodiment, the preferred vehicle for delivery of the cadherin-11 antagonist of the invention is a biocompatible microparticle or implant that is suitable for implantation into or in the vicinity of an afflicted tissue or organ in the recipient. Exemplary bioerodible implants that are useful in accordance with this method are described in WO 95/24929, entitled "Polymeric Gene Delivery System", claiming priority to U.S. patent application serial no. 213,668, filed March 15, 1994). WO95/24929 describes a biocompatible, preferably biodegradable polymeric matrix for containing an exogenous gene under the control of an appropriate promoter. The polymeric matrix is used to achieve sustained release of the exogenous gene in the subject. In accordance with the instant invention, the cadherin-11 antagonists described herein are encapsulated or dispersed within the biocompatible, preferably biodegradable polymeric matrix disclosed in WO95/24929. The polymeric matrix preferably is in the form of a microparticle such as a microsphere (wherein, for example, the cadherin-11 antagonist is dispersed throughout a solid polymeric matrix) or a microcapsule (wherein, for example, the cadherin-11 antagonist is stored in the core of a polymeric shell). Other forms of the polymeric matrix for containing the cadherin-11 antagonist include films, coatings, gels, implants, and stents. The size and composition of the polymeric matrix device is selected to result in favorable release kinetics in the tissue into which the matrix device is implanted. The size of the polymeric matrix devise is further selected according to the method of delivery which is to be used, typically injection into a tissue, or administration of a suspension by aerosol into the nasal and/or pulmonary areas. The polymeric matrix composition can be selected to have both favorable degradation rates and also to be formed of a material which is bioadhesive, to further increase the effectiveness of transfer when the devise is administered to a particular location in the body. The matrix composition also can be selected not to degrade, but rather, to release by diffusion over an extended period of time.

Both non-biodegradable and biodegradable polymeric matrices can be used to deliver the cadherin-11 antagonists. Biodegradable matrices are preferred. Such polymers may be natural or synthetic polymers. Synthetic polymers are preferred. The polymer is selected based on the period of time over which release is desired, generally in the order of a few hours to a year or longer. Typically, release over a period ranging from between a few hours and three to twelve months is most desirable. The polymer optionally is in the form of a hydrogel that can absorb up to about 90% of its weight in water and further, optionally is cross-linked with multi-valent ions or other polymers.

In general, the cadherin-11 antagonists of the invention are delivered using the bioerodible implant by way of diffusion, or more preferably, by degradation of the polymeric matrix. Exemplary synthetic polymers which can be used to form the biodegradable delivery system include: polyamides, polycarbonates, polyalkylenes, polyalkylene glycols, oxides, polyalkylene terepthalates, polyvinyl alcohols, polyvinyl ethers, polyvinyl esters, poly-vinyl halides, polyvinylpyrrolidone, polyglycolides, polysiloxanes, polyurethanes and co-polymers thereof, alkyl cellulose, hydroxyalkyl celluloses, cellulose ethers, cellulose esters, nitro celluloses, polymers of acrylic and methacrylic esters, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, carboxylethyl cellulose, cellulose triacetate, cellulose sulphate sodium salt, poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), polyethylene, polypropylene, poly(ethylene glycol), poly(ethylene oxide), poly (ethylene terephthalate), poly(vinyl alcohols), polyvinyl acetate, poly vinyl chloride, polystyrene and polyvinylpyrrolidone.

Examples of biodegradable polymers include synthetic polymers such as polymers of lactic acid and glycolic acid, polyanhydrides, poly(ortho)esters, polyurethanes, poly(butic acid), poly(valeric acid), and poly(lactide-co-caprolactone), and natural polymers such as alginate and other polysaccharides including dextran and cellulose, collagen, chemical derivatives thereof (substitutions, additions of chemical groups, for example, alkyl, alkylene, hydroxylations, oxidations, and other modifications routinely made by those skilled in the art), albumin and other hydrophilic proteins, zein and other prolamines and hydrophobic proteins, copolymers and mixtures thereof. In general, these materials degrade either by enzymatic hydrolysis or exposure to water *in vivo,* by surface or bulk erosion.

Bioadhesive polymers of particular interest include bioerodible hydrogels (described by H.S. Sawhney, C.P. Pathak and J.A. Hubell in Macromolecules, 1993, 26, 581-587, the teachings of which are incorporated herein), polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate).

Examples of non-biodegradable polymers include ethylene vinyl acetate, poly(meth)acrylic acid, polyamides, copolymers and mixtures thereof.

Other delivery systems can include time-release, delayed release or sustained release delivery systems. Such systems can avoid repeated administrations of the cadherin-11 antagonists described above, increasing convenience to the subject and the physician. Many types of release delivery systems are available and known to those of ordinary skill in the art. They include the above-described polymeric systems, as well as polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Patent 5,075,109. Delivery systems also include non-polymer systems that are: lipids including sterols such as cholesterol, cholesterol esters and fatty acids or neutral fats such as mono- di- and triglycerides; hydrogel release systems; silastic systems; peptide based systems; wax coatings; compressed tablets using conventional binders and excipients; partially fused implants; and the like. Specific examples include, but are not limited to: (a) erosional systems in which the cadherin-11 antagonist is contained in a form within a matrix such as those described in U.S. Patent Nos. 4,452,775, 4,675,189 and 5,736,152 and (b) diffusional systems in which an active component permeates at a controlled rate from a polymer such as described in U.S. Patent Nos. 3,854,480, 5,133,974 and 5,407,686. In addition, pump-based hardware delivery systems can be used, some of which are adapted for implantation.

Use of a long-term sustained release implant may be particularly suitable for treatment of chronic conditions. Long-term release, are used herein, means that the implant is constructed and arranged to delivery therapeutic levels of the active ingredient for at least 30 days, and preferably 60 days. Long-term sustained release implants are well-known to those of ordinary skill in the art and include some of the release systems described above.

The cadherin-11 antagonists can also be used, for example, to target a toxin (e.g., ricin) or a detectable agent (e.g., a radiolabel, a fluorescent label, an enzyme label) to cells which express cadherin-11 counter-receptors or cadherin-11. Methods for coupling such toxins and/or agents to proteins and/or antibodies for in vivo and in vitro applications are disclosed in, for example, Killen and Lindstrom (1984), "Specific killing of lymphocytes that cause experimental Autoimmune Myestenia Gravis by toxin-acetylcholine receptor conjugates", J. Immun. 133:1335; Jansen, F.K., et al. (1982), "Immunotoxins: Hybrid molecules combining high specificity and potent cytotoxicity", Immunolog. Rev. 62:185 -216. See also U.S. Patent Nos. 3,652,761; 4,478,946 and 4,554,088.

The invention will be more fully understood by reference to the following examples. These examples, however, are merely intended to illustrate the embodiments of the invention and are not to be construed to limit the scope of the invention. It is also to be understood that the reference figures are illustrative only and are not essential to the enablement of the claimed invention.

### EXAMPLES

### Example 1: Treatment of atopic dermatitis by anti-cadherin-11 monoclonal antibody.

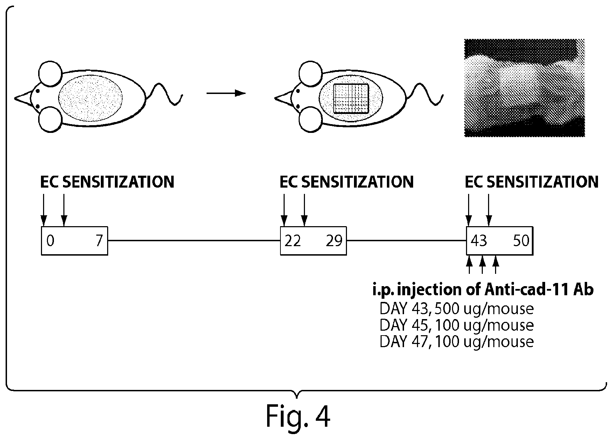

To determine whether cadherin-11 modulates skin inflammation, a mouse model of atopic dermatitis (AD) was used. In this model, skin inflammation displays many features of human AD or eczema which is a chronically relapsing inflammatory skin disorder. A hallmark of AD is dry itchy skin. Scratching causes skin injury and worsens the disease symptoms. Therefore, the model mimics skin irritation by first shaving the back of the mouse and then applying and removing tegaderm tape several times from the same shaved area. Mice are then epicutaneously (EC) sensitized with saline (SAL) or ovalbumin (OVA) (100 µg/100 µl of saline) soaked gauze which is applied to the tape-stripped area at days 0, 3, 22, 25, 43, and 46. The gauze is placed on the back skin for one week periods as shown in FIG. 4. Chronic skin inflammation develops by repeating the procedure a total of three times at two weeks intervals (FIG. 4).

To determine the effect of blocking cadherin-11 on this skin inflammation model, we treated mice with anti-cadherin-11 (cad-11) monoclonal antibody (mAb) during the third cycle as shown in FIG. 4. mAb was i.p. injected at days 43, 45, and 47. Mice were sacrificed and examined at day 50. For comparison, after the last tape adhesion and removal, skin tissues were collected to compare the immune responses against OVA between untreated and anti-cadherin-11 mAb treated mice.

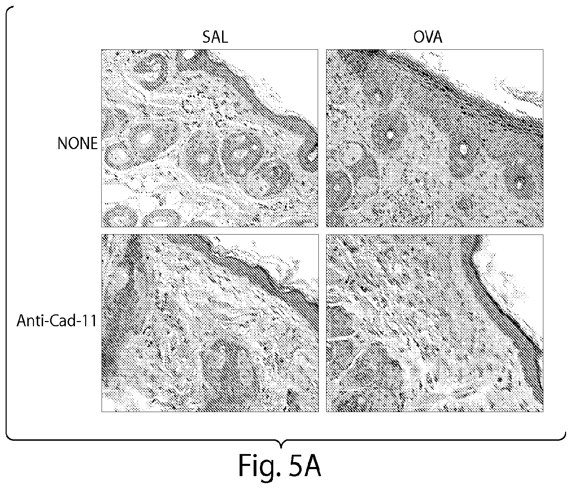

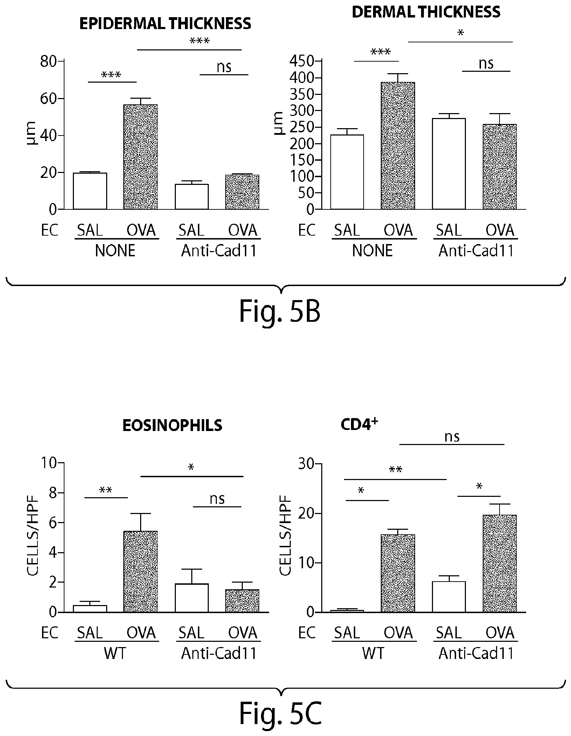

Anti-cadherin-11 mAb treatment significantly reduced epidermal and dermal thickness in H&E staining of sections from control (saline, SAL) or OVA- sensitized skin (FIGs. 5A and 5B). Although the total number of CD4⁺ T cells recruited the lesions was not significantly changed in anti-cadherin-11 mAb treated mice, eosinophil recruitment was significantly decreased in anti-cadherin-11 mAb-treated mice compared to untreated mice (FIG. 5C). Eosinophils are another key effector cell type. Consistent with this, the level of eotaxin (a key chemokine for eosinophils) was reduced in OVA-sensitized skin from anti-cadherin-11 mAb treated mice.

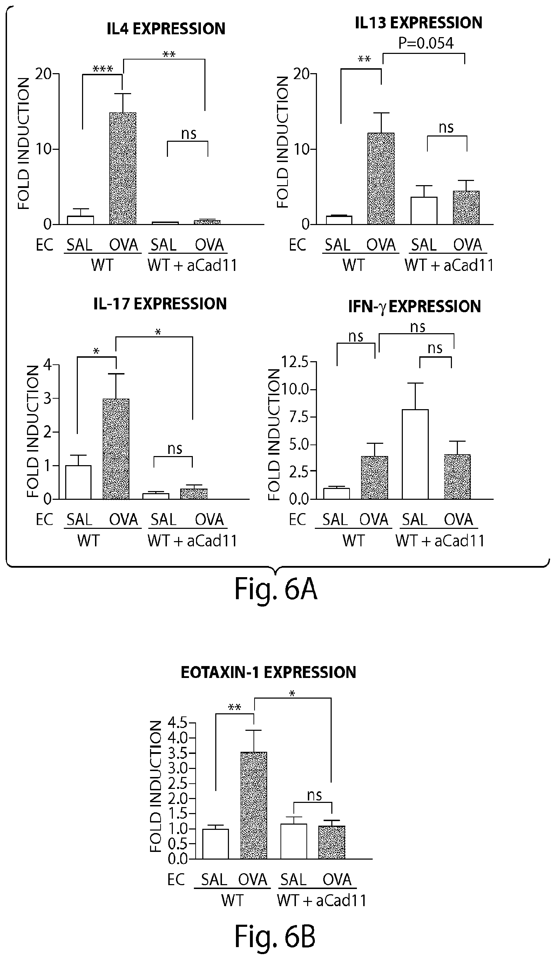

Since the pathology of this mouse model of AD is usually dominated by Th2 and/or Th17 immune responses, we determined if anti-cadherin-11 mAb treatment modifies the immune responses in the affected skin. We detected Th2 type cytokines (IL-4 and IL-13), Th17 type cytokine (IL-17), and Th1 type cytokine (IFN-γ) by quantitative real time PCR in the saline (SAL)- or ovalbumin (OVA)- sensitized skin from control or anti-cadherin-11 mAb-treated mice. Strikingly, there was significantly less production of both IL-4 and IL-17 in the affected skin from anti-cadherin-11 mAb treated mice compared to control mice (FIG. 6A). The production of IL-13 also showed a similar pattern as IL-4 or IL-17 (FIG. 6A). In contrast, the level of IFN-γ (Th1 type cytokine) was not changed by anti-cadherin-11 mAb treatment (FIG. 6A). Thus, anti-cadherin-11 mAb treatment blocked the Th2 and/or Th17-immune responses against antigen in the sensitized skin.

These *in vivo* data indicate that anti-cadherin-11 mAb treatment reduces local allergic skin inflammation.

### Example 2: Cadherin-11 deficient mice are protected from colitis in an experimentally induced model of inflammatory bowel disease.

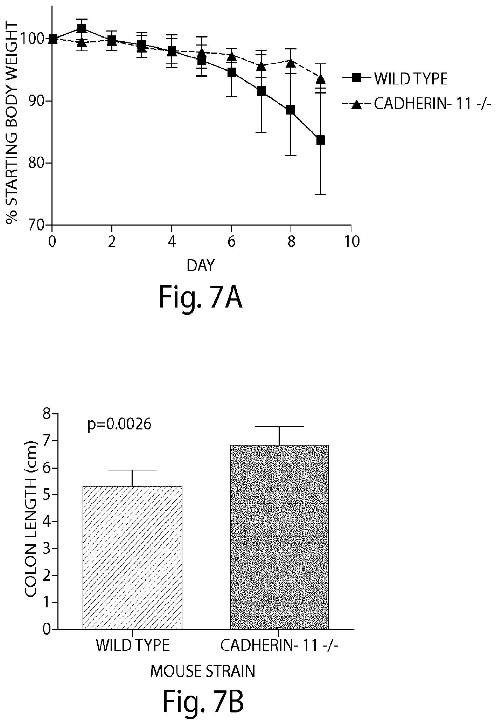

The role of cadherin-11 in inflammatory bowel disease was analyzed using a dextran sodium sulfate induced inflammatory bowel disease mouse model. Wildtype and cadherin-11-deficient (cad-11^{-/-}) mice ingested 2% dextran sodium sulfate in their drinking water *ad libitum* for days 0 through 6 (n=6 mice per group). The development of colitis was followed by the severity of weight loss over time, expressed as a change from starting body weight (FIG. 7A). Cadherin-11^{-/-} mice do not lose weight compared to wildtype mice indicating that colitis is less severe in cad-11^{-/-} mice. In addition, on day 9, mice were sacrificed to measure colon length (FIG. 7B). Shorter colon lengths correlate with more severe colitis. The longer length in cad-11^{-/-} mice reflects less colonic disease. These results show that cadherin-11 deficient mice to not develop severe colitis compared to wild type mice in this model system. Cadherin-11 inhibition, for example via anti-cadherin-11 antibodies, is expected to also interfere with the development (or maintenance) of inflammatory bowel diseases such as colitis.

### EQUIVALENTS

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the disclosures described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings is/are used. Those skilled in the art will recognize, or be able to ascertain using no more than routine experimentation, many equivalents to the specific embodiments described herein. Embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the present disclosure.

All definitions, as defined and used herein, should be understood to control over dictionary definitions, definitions in documents cited herein and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03.

## Claims

1. A cadherin-11 antagonist in a therapeutically effective amount for use in a method for treating a subject having a non-joint inflammatory disorder, wherein the subject does not have fibrosis, and
wherein the cadherin-11 antagonist is an anti-cadherin-11 antibody or an antigen-binding antibody fragment thereof or wherein the cadherin-11 antagonist is a nucleic acid that binds to cadherin-11 or a cadherin-11 nucleic acid.

2. A cadherin-11 antagonist for use according to claim 1, wherein the disorder is inflammatory bowel disease.

3. A cadherin-11 antagonist for use according to claim 2, wherein the antagonist is administered to the gut.

4. A cadherin-11 antagonist for use according to claim 1, wherein the disorder is atopic dermatitis.

5. A cadherin-11 antagonist for use according to claim 4, wherein the antagonist is administered to the skin.

6. A cadherin-11 antagonist for use according to any one of claims 1 to 5, wherein the nucleic acid cadherin-11 antagonist is:
(a) a cadherin-11 siRNA;
(b) a cadherin-11 ribozyme;
(c) a cadherin-11 antisense molecule;
(d) a cadherin-11 RNAi; or
(e) an aptamer.

7. A cadherin-11 antagonist for use according to any of claims 1-6, wherein the method further comprises administering to the subject an immunosuppressant.

8. A cadherin-11 antagonist for use according to claim 7, wherein the immunosuppressant is a steroid.

9. A cadherin-11 antagonist for use according to any of claims 1-8, wherein the anti-cadherin-11 antibody or antigen-binding antibody fragment thereof is a monoclonal anti-cadherin-11 antibody or anti-cadherin-11 antibody fragment.

10. A cadherin-11 antagonist for use according to claim 9, wherein the anti-cadherin-11 antibody or antigen-binding antibody fragment thereof is a human anti-cadherin-11 antibody or anti-cadherin-11 antibody fragment.

11. A cadherin-11 antagonist for use according to any of claims 1-10, wherein the cadherin-11 antagonist is administered parenterally to the subject.

12. A cadherin-11 antagonist for use according to any of claims 1-10, wherein the cadherin-11 antagonist is administered intravenously to the subject.

13. A cadherin-11 antagonist for use according to any of claims 1-12, wherein the subject is a human subject.

14. A cadherin-11 antagonist for use according to any of claims 1-13, wherein the therapeutically effective amount is an amount effective to reduce inflammation.

## Patentansprüche

1. Cadherin-11-Antagonist in einer therapeutisch wirksamen Menge zur Verwendung in einem Verfahren zur Behandlung eines Individuums mit einer nichtgelenkbezogenen Entzündungserkrankung, wobei das Individuum keine Fibrose aufweist, und
wobei der Cadherin-11-Antagonist ein anti-Cadherin-11-Antikörper oder ein Antigen-bindendes Antikörperfragment davon ist oder wobei der Cadherin-11-Antagonist eine Nukleinsäure ist, die an Cadherin-11 oder eine Cadherin-11-Nukleinsäure bindet.

2. Cadherin-11-Antagonist zur Verwendung nach Anspruch 1, wobei die Erkrankung eine entzündliche Darmerkrankung ist.

3. Cadherin-11-Antagonist zur Verwendung nach Anspruch 2, wobei der Antagonist an den Darm verabreicht wird.

4. Cadherin-11-Antagonist zur Verwendung nach Anspruch 1, wobei die Erkrankung atopische Dermatitis ist.

5. Cadherin-11-Antagonist zur Verwendung nach Anspruch 4, wobei der Antagonist an die Haut verabreicht wird.

6. Cadherin-11-Antagonist zur Verwendung nach einem der Ansprüche 1 bis 5, wobei der Nukleinsäure-Cadherin-11-Antagonist
(a) eine Cadherin-11-siRNA;
(b) ein Cadherin-11-Ribozym;
(c) ein Cadherin-11 Antisense-Molekül;
(d) ein Cadherin-11-RNAi; oder
(e) ein Aptamer
ist.

7. Cadherin-11-Antagonist zur Verwendung nach einem der Ansprüche 1 - 6, wobei das Verfahren weiterhin die Verabreichung eines Immunsuppressivums an das Individuum umfasst.

8. Cadherin-11-Antagonist zur Verwendung nach Anspruch 7, wobei das Immunsuppressivum ein Steroid ist.

9. Cadherin-11-Antagonist zur Verwendung nach einem der Ansprüche 1 - 8, wobei der anti-Cadherin-11-Antikörper oder das Antigen-bindende Antikörperfragment davon ein monoklonaler anti-Cadherin-11-Antikörper oder anti-Cadherin-11-Antikörperfragment ist.

10. Cadherin-11-Antagonist zur Verwendung nach Anspruch 9, wobei der anti-Cadherin-11-Antikörper oder das Antigen-bindende Antikörperfragment davon ein humaner anti-Cadherin-11-Antikörper oder anti-Cadherin-11-Antikörperfragment ist.

11. Cadherin-11-Antagonist zur Verwendung nach einem der Ansprüche 1 - 10, wobei der Cadherin-11-Antagonist parenteral an das Individuum verabreicht wird.

12. Cadherin-11-Antagonist zur Verwendung nach einem der Ansprüche 1 - 10, wobei der Cadherin-11-Antagonist intravenös an das Individuum verabreicht wird.

13. Cadherin-11-Antagonist zur Verwendung nach einem der Ansprüche 1 - 12, wobei das Individuum ein humanes Individuum ist.

14. Cadherin-11-Antagonist zur Verwendung nach einem der Ansprüche 1 - 13, wobei die therapeutisch wirksame Menge eine Menge ist, die wirksam ist, um Entzündung zu reduzieren.

## Revendications

1. Antagoniste de la cadhérine-11 en une quantité thérapeutiquement efficace, pour utilisation dans un procédé de traitement d'un sujet souffrant d'un trouble inflammatoire non articulaire, où le sujet ne souffre pas de fibrose, et
où l'antagoniste de la cadhérine-11 est un anticorps anti-cadhérine-11 ou un de ses fragments d'anticorps se liant à l'antigène ou où l'antagoniste de la cadhérine-11 est un acide nucléique qui se lie à la cadhérine-11 ou à un acide nucléique de la cadhérine-11.

2. Antagoniste de la cadhérine-11 pour utilisation selon la revendication 1, où le trouble est une maladie inflammatoire de l'intestin.

3. Antagoniste de la cadhérine-11 pour utilisation selon la revendication 2, où l'antagoniste est administré à l'intestin.

4. Antagoniste de la cadhérine-11 pour utilisation selon la revendication 1, où le trouble est la dermatite atopique.

5. Antagoniste de la cadhérine-11 pour utilisation selon la revendication 4, où l'antagoniste est administré à la peau.

6. Antagoniste de la cadhérine-11 pour utilisation selon l'une quelconque des revendications 1 à 5, où l'antagoniste de la cadhérine-11 de type acide nucléique est :
(a) un ARNsi de la cadhérine-11 ;
(b) un ribozyme de la cadhérine-11 ;
(c) une molécule antisens de la cadhérine-11 ;
(d) un ARNi de la cadhérine-11 ; ou
(e) un aptamère.

7. Antagoniste de la cadhérine-11 pour utilisation selon l'une des revendications 1 à 6, où le procédé comprend en outre l'étape consistant à administrer au sujet un immunosuppresseur.

8. Antagoniste de la cadhérine-11 pour utilisation selon la revendication 7, où l'immunosuppresseur est un stéroïde.

9. Antagoniste de la cadhérine-11 pour utilisation selon l'une des revendications 1 à 8, dans lequel l'anticorps anti-cadhérine-11 ou un des ses fragments d'anticorps se liant à l'antigène est un anticorps monoclonal anti-cadhérine-11 ou un fragment d'anticorps monoclonal anti-cadhérine-11.

10. Antagoniste de la cadhérine-11 pour utilisation selon la revendication 9, dans lequel l'anticorps anti-cadhérine-11 ou un des ses fragments d'anticorps se liant à l'antigène est un anticorps anti-cadhérine-11 humain ou un fragment d'anticorps anti-cadhérine-11 humain.

11. Antagoniste de la cadhérine-11 pour utilisation selon l'une des revendications 1 à 10, où l'antagoniste de la cadhérine-11 est administré par voie parentérale au sujet.

12. Antagoniste de la cadhérine-11 pour utilisation selon l'une des revendications 1 à 10, où l'antagoniste de la cadhérine-11 est administré par voie intraveineuse au sujet.

13. Antagoniste de la cadhérine-11 pour utilisation selon l'une des revendications 1 à 12, où le sujet est un sujet humain.

14. Antagoniste de la cadhérine-11 pour utilisation selon l'une des revendications 1 à 13, dans lequel la quantité thérapeutiquement efficace est une quantité efficace pour réduire l'inflammation.
